# EUROPEAN PATENT APPLICATION

(11) **EP 3 932 405 A1**
(43) Date of publication of application: **05.01.2022**
(21) Application number: 20762674.8
(22) Date of filing: 24.01.2020
(51) Int. Cl.: A61K 31/425, A61K 31/443, A61K 31/4436, A61P 25/00, A61P 25/16, A61P 25/28, C07D 405/06, C07D 417/06, A61K 51/04

(54) **alpha-SYNUCLEIN AGGREGATE BINDING AGENT AND IMAGING METHOD**

(30) Priority: 27.02.2019 JP 2019034997
(71) Applicant: NATIONAL INSTITUTES FOR QUANTUM AND RADIOLOGICAL SCIENCE AND TECHNOLOGY, Inage-ku Chiba-shi Chiba 263-8555 (JP)
(72) Inventor: HIGUCHI Makoto, Chiba-shi, Chiba 263-8555 (JP); ONO Maiko, Chiba-shi, Chiba 263-8555 (JP); SUHARA Tetsuya, Chiba-shi, Chiba 263-8555 (JP); CHO Meiei, Chiba-shi, Chiba 263-8555 (JP)
(74) Representative: Plasseraud IP
(86) International application number: PCT/JP2020/002607
(87) International publication number: WO 2020/174963

(57) **Abstract**

The present invention provides an α-synuclein aggregate binding agent that has high binding selectivity for an α-synuclein aggregate.

The α-synuclein aggregate binding agent contains a compound represented by a formula (I), a pharmaceutically acceptable salt thereof, or a solvate thereof: 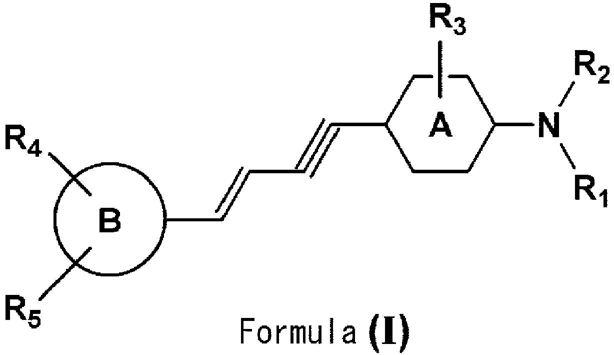 in the formula (I), R₁ and R₂ are each independently selected from the group consisting of hydrogen, alkyl, alkenyl, acyl, and hydroxyalkyl; R₃ is hydrogen or halogen; the ring A is a benzene or pyridine ring; the ring B is represented by the following formula (i) or (ii): 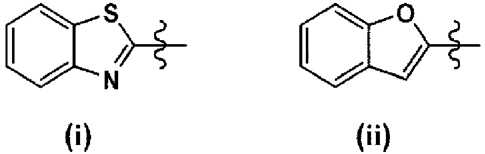 R₄ and R₅ are each independently selected from the group consisting of hydrogen, hydroxy, alkoxy, haloalkoxy, halohydroxyalkoxy, and aminoalkyl.

## Description

### Technical Field

The present invention relates to an α-synuclein aggregate binding agent, a composition for optical imaging of an α-synuclein aggregate, a composition for radiological imaging of an α-synuclein aggregate, a diagnostic agent for a disease associated with an α-synuclein aggregate, a companion diagnostic agent for treating or preventing the disease, a diagnostic kit for a disease associated with a substance accumulated in the brain, an optical imaging method, a radiological imaging method, a method of screening for a therapeutic or preventive agent for a disease associated with an α-synuclein aggregate in the brain, a method for carrying out quantification or determination of accumulation of an α-synuclein aggregate in the brain, a method for classification and detection of a substance accumulated in the brain, an α-synuclein aggregation inhibitor, and a novel compound.

### Background Art

An α-synuclein aggregate forms a core pathology of Parkinson's disease, dementia with Lewy bodies (DLB), and multiple system atrophy (MSA) and is considered to have a close causal association with neurodegeneration. Definitive diagnosis of these diseases is carried out while using, as an indicator, the presence of an α-synuclein aggregate in a neuropathological examination of the autopsied brain. Therefore, it is impossible to make the definitive diagnosis while the patient is alive. However, if the α-synuclein aggregate can be visualized in the living brain, it is possible to obtain information close to the definitive diagnosis of those diseases from an early stage. Moreover, if the α-synuclein aggregate can be visualized in the brain of a living disease model animal, such visualization can also help assess efficacy of a candidate substance for a therapeutic or preventive agent targeting the α-synuclein aggregate, by imaging over time and the like.

[¹¹C]BF-227 is an example of a positron emission tomography (PET) probe that has previously shown to bind to α-synuclein in the living brain. However, [¹¹C]BF-227 has insufficient binding affinity for the α-synuclein aggregate and, among those diseases, α-synuclein lesions can be detected only in a subset of MSA patients. In addition, [¹¹C]BF-227 has a problem of nonspecific accumulation in the brain and a problem of low binding selectivity for the α-synuclein aggregate because [¹¹C]BF-227 binds to an amyloid-β aggregate.

The inventors have developed a novel compound for imaging tau protein accumulated in the brain (see Patent Literature 1). The compound disclosed in Patent Literature 1 allows imaging of tau proteins accumulated in the brain, and therefore the technique of Patent Literature 1 is useful for treatment, prevention, and the like of a disease (e.g., Alzheimer's disease (AD)) caused by accumulation of tau proteins. However, Patent Literature 1 does not disclose binding to the α-synuclein aggregate.

### Citation List

### [Patent Literature]

[Patent Literature 1] International Publication No. WO 2014/097474, pamphlet

### Summary of Invention

### Technical Problem

Among the compounds disclosed in Patent Literature 1, the present inventors have found that (2-((1E,3E)-4-(6-(methylamino)pyridin-3-yl)buta-1,3-dienyl)benzo[d]thiazol-6-ol (PBB3) can also bind to the α-synuclein aggregate with some degree of affinity.

However, PBB3 has lower binding selectivity for the α-synuclein aggregate as compared with its binding selectivity for a tau aggregate. Therefore, PBB3 has low compatibility as an α-synuclein aggregate probe. In this context, an α-synuclein aggregate binding agent having high binding selectivity for the α-synuclein aggregate is demanded.

If it is possible, not only to carry out diagnosis by detecting the α-synuclein aggregate, but also to inhibit (control) the α-synuclein aggregate itself, diseases derived from the α-synuclein aggregate can be prevented or treated. Therefore, an α-synuclein aggregation inhibitor capable of inhibiting aggregation of α-synuclein is demanded.

The present invention is accomplished in view of the above circumstances, and its objective is to provide an α-synuclein aggregate binding agent having high binding selectivity for an α-synuclein aggregate, an imaging method using the α-synuclein aggregate binding agent, a novel compound which can be used as an α-synuclein aggregate binding agent, and an α-synuclein aggregation inhibitor.

### Solution to Problem

The present inventors have found that, among the compounds disclosed in Patent Literature 1, a compound represented by the following formula (I) having a buteninyl structure and a benzothiazole or benzofuran structure has high binding selectivity for the α-synuclein aggregate, and further studied to accomplish the present invention. More specifically, the present invention provides the following features.

[1] An α-synuclein aggregate binding agent containing a compound represented by the following formula (I), a pharmaceutically acceptable salt thereof, or a solvate thereof: 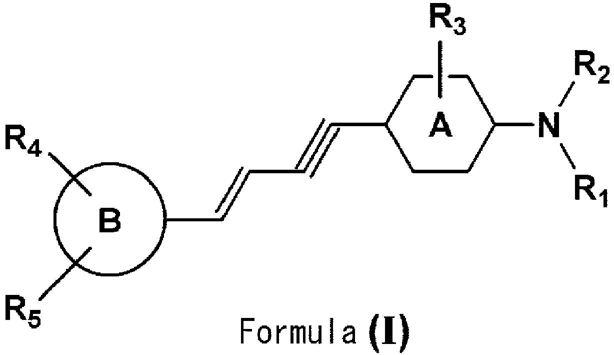 in the formula (I),
   R₁ and R₂ are each independently selected from the group consisting of hydrogen, alkyl, alkenyl, acyl, and hydroxyalkyl;
   R₃ is hydrogen or halogen;
   the ring A is a benzene or pyridine ring;
   the ring B is represented by the following formula (i) or (ii): 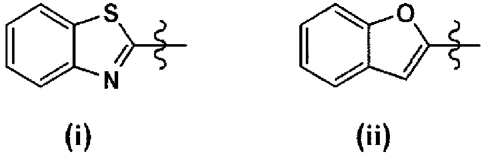
   R₄ and R₅ are each independently selected from the group consisting of hydrogen, hydroxy, alkoxy, haloalkoxy, halohydroxyalkoxy, and aminoalkyl.
[2] The binding agent described in [1], in which the ring A is a pyridine ring.
[3] The binding agent described in [1] or [2], in which the ring B is represented by the formula (i).
[4] The binding agent described in [1], in which the compound represented by the formula (I) is selected from the following group: 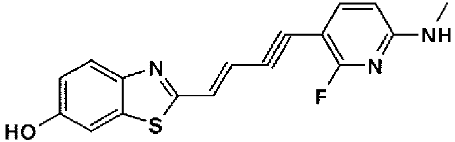 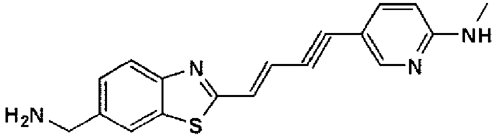 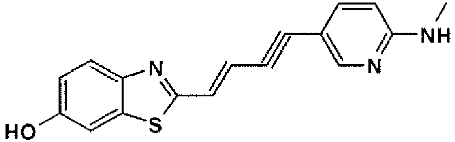 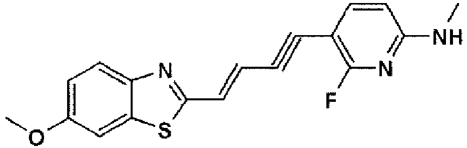 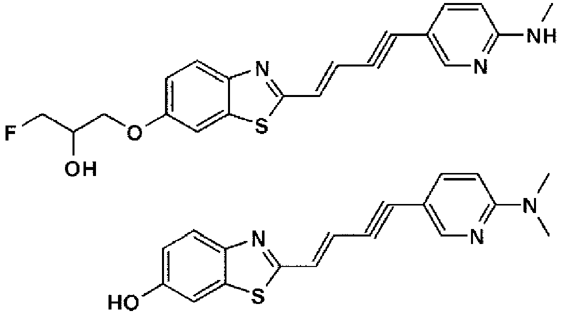 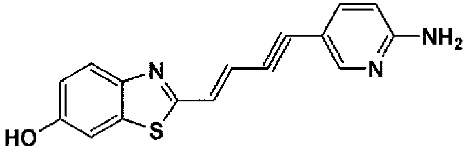 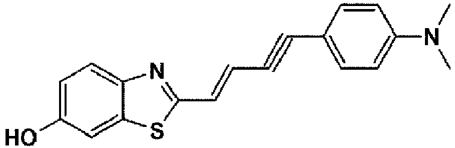 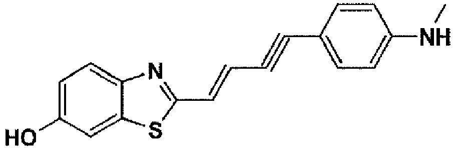 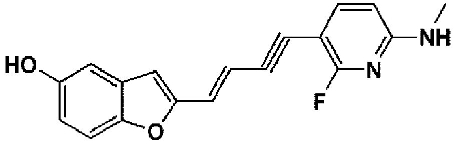 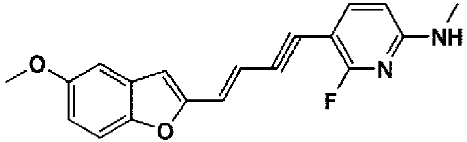
[5] The binding agent described in any of [1] through [4], in which, in the compound represented by the formula (I), one or more atoms are radioisotopes thereof.
[6] A composition for optical imaging of an α-synuclein aggregate, the composition containing the binding agent described in any of [1] through [4].
[7] A composition for radiological imaging of an a-synuclein aggregate, the composition containing the binding agent described in [5].
[8] A diagnostic agent for a disease associated with an α-synuclein aggregate or a companion diagnostic agent for treating or preventing the disease, containing the binding agent described in any of [1] through [5].
[9] A diagnostic kit for a disease associated with a substance accumulated in the brain, the diagnostic kit including: the binding agent described in any of [1] through [5]; and at least one compound selected from 2-((1E,3E)-4-(6-(methylamino)pyridin-3-yl)buta-1,3-dienyl)benzo[d]thiazol-6-ol, 2-((1E,3E)-4-(6-((11)C-methylamino)pyridin-3-yl)buta-1,3-dienyl)benzo[d]thiazol-6-ol, 1-fluoro-3-(2-((1E,3E)-4-(6-(methylamino)pyridin-3-yl)buta-1,3-dienyl)benzo [d]thiazol-6-yloxy)propan-2-ol, and 1-(18)F-fluoro-3-(2-((1E,3E)-4-(6-(methylamino)pyridin-3-yl)buta-1,3-dienyl)benzo[d]thiazol-6-yloxy)propan-2-ol.
[10] A method for carrying out optical imaging of an α-synuclein aggregate in the brain, the method including the step of: externally irradiating the brain of a living subject, to which the binding agent described in any of [1] through [4] has been administered, with light having the first wavelength, and then detecting light which is emitted from the brain and has the second wavelength different from the first wavelength.
[11] A method for carrying out radiological imaging of an α-synuclein aggregate in the brain, the method including the step of: detecting radioactivity that is emitted from the brain of a living subject to which the binding agent described in [5] has been administered.
[12] A method of screening for a therapeutic or preventive agent for a disease associated with an α-synuclein aggregate in the brain,
   the method including the step of: selecting a candidate substance based on a difference, which has been caused by administration of the candidate substance to the subject, in quantity and/or distribution of light or radioactivity which is detected in the method described in [10] or [11].
[13] A method for carrying out quantification or determination of accumulation of an α-synuclein aggregate in the brain, the method including the step of: detecting radioactivity that is emitted from the brain of a living subject to which the binding agent described in [5] has been administered, the quantification or determination being carried out based on a quantity and/or distribution of the radioactivity which has been detected.
[14] A method for classification and detection of a substance accumulated in the brain, the method including:
   the first step of detecting radioactivity that is emitted from the brain of a living subject to which the binding agent described in [5] has been administered; and
   the second step of detecting radioactivity that is emitted from the brain of the subject to which at least one compound selected from 2-((1E,3E)-4-(6-((11)C-methylamino)pyridin-3-yl)buta-1,3-dienyl)benzo[d]thiazol-6-ol and 1-(18)F-fluoro-3-(2-((1E,3E)-4-(6-(methylamino)pyridin-3-yl)buta-1,3-dienyl)benzo[d]thiazol-6-yloxy)propan-2-ol has been administered at a time point which is different from that of the first step,
   the determination being carried out based on data of a quantity and/or distribution of the radioactivity detected in the first step and on data of a quantity and/or distribution of the radioactivity detected in the second step.
[15] An α-synuclein aggregation inhibitor containing a compound represented by the following formula (I), a pharmaceutically acceptable salt thereof, or a solvate thereof: 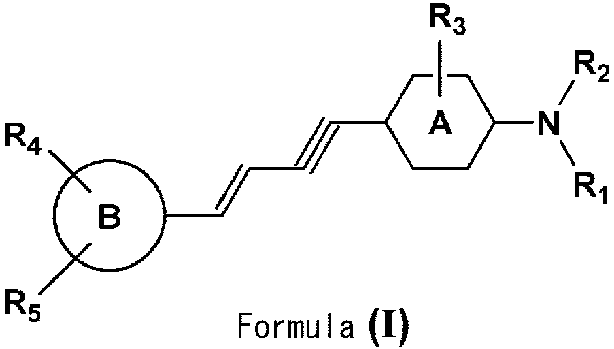 in the formula (I),
   R₁ and R₂ are each independently selected from the group consisting of hydrogen, alkyl, alkenyl, acyl, and hydroxyalkyl;
   R₃ is hydrogen or halogen;
   the ring A is a benzene or pyridine ring;
   the ring B is represented by the following formula (i) or (ii): 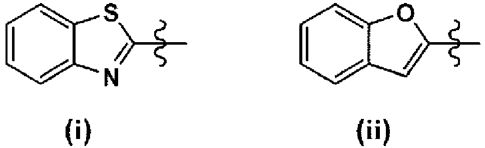
   R₄ and R₅ are each independently selected from the group consisting of hydrogen, hydroxy, alkoxy, haloalkoxy, halohydroxyalkoxy, and aminoalkyl.
[16] The α-synuclein aggregation inhibitor described in [15], in which the ring A is a pyridine ring.
[17] The α-synuclein aggregation inhibitor described in [15] or [16], in which the ring B is represented by the formula (i).
[18] The α-synuclein aggregation inhibitor described in [15], in which the compound represented by the formula (I) is selected from the following group: 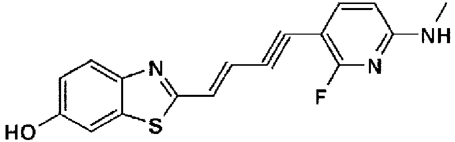 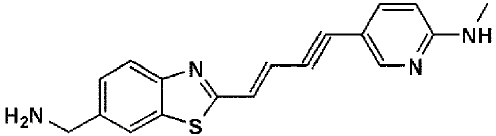 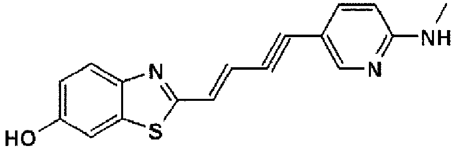 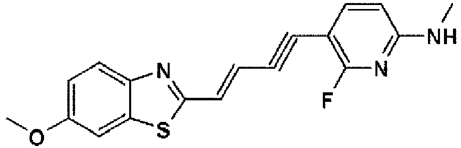 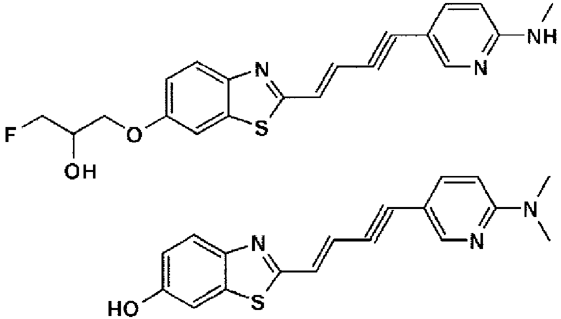 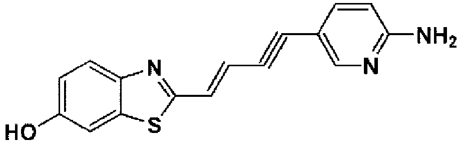 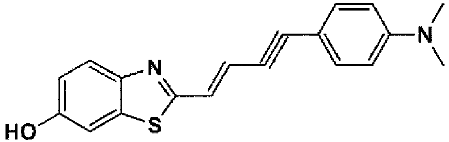 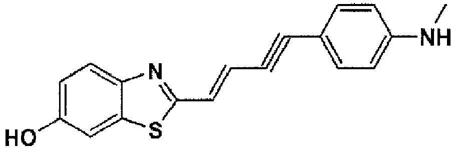 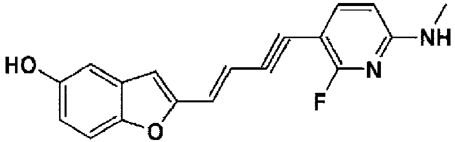 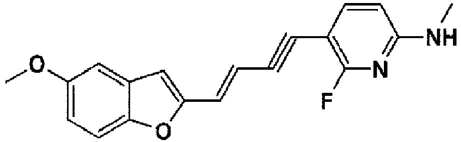
[19] A compound represented by the following formula, a pharmaceutically acceptable salt thereof, or a solvate thereof: 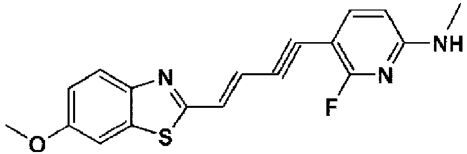 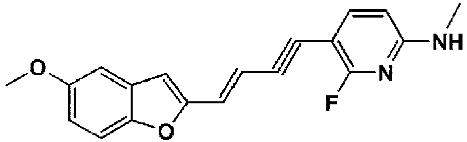
[20] A compound represented by the following formula, a pharmaceutically acceptable salt thereof, or a solvate thereof: 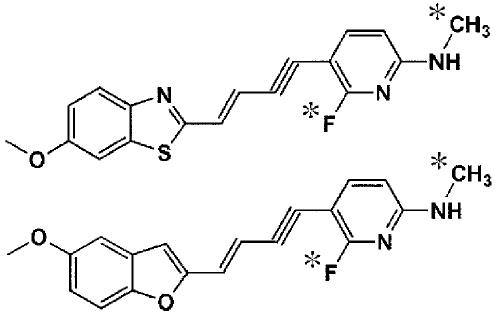 wherein at least one of atoms with the symbol * is a radioisotope thereof.
[21] An intermediate for synthesizing the compound described in [19], in which the intermediate is represented by the following formula: 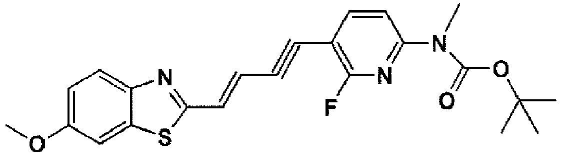 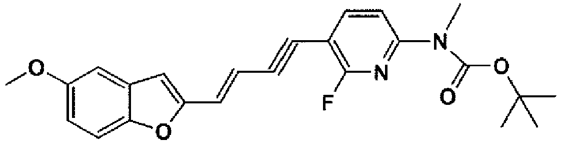
[22] An intermediate for synthesizing a compound described in [20], in which the intermediate is represented by the following formula:

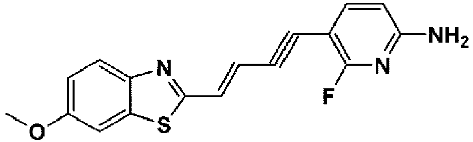

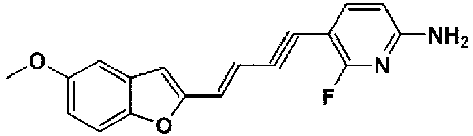

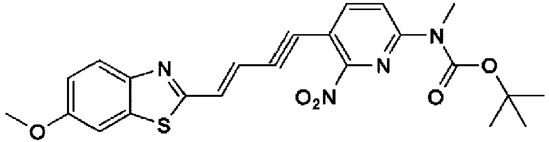

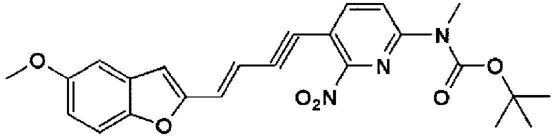

### Advantageous Effects of Invention

According to the present invention, it is possible to provide the α-synuclein aggregate binding agent that has high binding selectivity for an α-synuclein aggregate.

Further, it is possible to provide the imaging method using the α-synuclein aggregate binding agent.

Moreover, it is possible to provide the novel compound that can be used as an α-synuclein aggregate binding agent or can be used for other applications.

Furthermore, it is possible to inhibit aggregation of α-synuclein.

### Brief Description of Drawings

Fig. 1A is a diagram showing results of fluorescence microscope measurement of the brains of DLB and AD patients.
Fig. 1B is a diagram showing results of fluorescence microscope measurement of the brains of DLB and AD patients.
Fig. 2 is a diagram showing results of quantifying fluorescence in areas with and without the formation of lesions.
Fig. 3 is a diagram showing results of fluorescence microscope measurement of an α-synuclein fibril-injected mouse.
Fig. 4 is a diagram showing results of two-photon laser scanning fluorescence microscopy of a model mouse 6 weeks after injection of an α-synuclein fiber solution.
Fig. 5 is a diagram showing results of PET imaging.
Fig. 6 is a diagram showing results of PET imaging.
Fig. 7 is a diagram showing results of fluorescence microscopy of an α-synuclein fibril-injected mouse.
Fig. 8 is a diagram showing results of PET imaging.
Fig. 9 is a diagram showing results of PET imaging.
Fig. 10 is a diagram showing results of PET imaging.
Fig. 11 is a diagram showing results of PET imaging.
Fig. 12 is a diagram showing results of PET imaging.
Fig. 13 is a diagram showing an effect of inhibiting α-synuclein aggregation.
Fig. 14 is a diagram showing an effect of inhibiting α-synuclein aggregation.

### Description of Embodiments

### <Definitions>

The term "alkyl" means a monovalent group that is obtained when one hydrogen atom is lost from aliphatic saturated hydrocarbon. Alkyl has, for example, 1 to 15 carbon atoms, typically, 1 to 10, 1 to 8, 1 to 6, 1 to 5, 1 to 4, 1 to 3, 1 to 2, or 2 to 6 carbon atoms. Alkyl can be in a linear chain form, a branched form, or a cyclic form. Examples of alkyl include, but not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, and the like. Alkyl can be further substituted by an appropriate substituent.

Herein, 1 to 15, 1 to 10, 1 to 8, 1 to 6, 1 to 5, 1 to 4, 1 to 3, 1 to 2, 2 to 8, 2 to 6, 2 to 4, 3 to 8, 3 to 6, 4 to 8, and 4 to 6 carbon atoms are also referred to as C₁₋₁₅, C₁₋₁₀, C₁₋₈, C₁₋₆, C₁₋₅, C₁₋₄, C₁₋₃, C₁₋₂, C₂₋₈, C₂₋₆, C₂₋₄, C₃₋₈, C₃₋₆, C₄₋₈, and C₄₋₆, respectively.

The term "cycloalkyl" means a monovalent group that is obtained when one hydrogen atom is lost from aliphatic saturated hydrocarbon forming a carbocyclic ring. Cycloalkyl has, for example, 3 to 10 carbon atoms, typically, 3 to 8, 3 to 6, 3 to 5, 3 to 4, 4 to 5, 4 to 6, or 4 to 8 carbon atoms. Examples of cycloalkyl include, but not limited to, cyclopropane, cyclobutane, cyclopentane, cyclohexane, cycloheptane, and cyclooctane. Cycloalkyl can be further substituted by an appropriate substituent.

The term "alkenyl" means an unsaturated aliphatic hydrocarbon group that has at least one double bond. Alkenyl has, for example, 2 to 15 carbon atoms, typically, 2 to 10, 2 to 8, 2 to 6, 2 to 5, 2 to 4, 2 to 3, 3 to 6, 3 to 8, 4 to 6, 4 to 7, or 4 to 8 carbon atoms. Alkenyl can be in a linear chain form or in a branched form. Specific examples of alkenyl include, but not limited to, vinyl (-CH=CH₂), allyl (-CH₂CH=CH₂), - CH=CH(CH₃), -CH=C(CH₃)₂, -C(CH₃)=CH₂, -C(CH₃)=CH(CH₃), - C(CH₂CH₃)=CH₂, 1,3-butadienyl (-CH=CH-CH=CH₂), hepta-1,6-diene-4-yl (-CH₂-(CH₂CH=CH₂)₂), and the like. Alkenyl can be further substituted by an appropriate substituent.

The term "alkynyl" means an unsaturated aliphatic hydrocarbon group that has at least one triple bond. Alkynyl has, for example, 2 to 15 carbon atoms, typically, 2 to 10, 2 to 8, 2 to 6, 2 to 5, 2 to 4, 2 to 3, 3 to 6, 4 to 6, 4 to 7, or 4 to 8 carbon atoms. Alkynyl can be in a linear chain form or in a branched form. Examples of alkynyl include, but not limited to, ethynyl (-C=CH), -C≡CH(CH₃), -C≡C(CH₂CH₃), -CH₂C≡CH, - CH₂C≡C(CH₃), -CH₂C≡C(CH₂CH₃), and the like. Alkynyl can be further substituted by an appropriate substituent.

The term "acyl" means a group that is represented by "-CO-R". Here, R is, for example, alkyl, alkenyl, or alkynyl. Examples of acyl include, but not limited to, acetyl (-COCH₃), ethylcarbonyl, propylcarbonyl, pentylcarbonyl, cyclohexylcarbonyl, octylcarbonyl, 2-ethylhexylcarbonyl, dodecylcarbonyl, phenylcarbonyl, benzylcarbonyl, naphthylcarbonyl, pyridylcarbonyl, and the like. Acyl can be further substituted by an appropriate substituent.

The term "hydroxy" or "hydroxyl" means -OH.

The term "hydroxyalkyl" means an alkyl group that is substituted by a hydroxy group (-OH). Examples of hydroxyalkyl include, but not limited to, hydroxymethyl (-CH₂OH), 2-hydroxyethyl (-CH₂CH₂OH), 1-hydroxyethyl (-CH(OH)CH₃), 3-hydroxypropyl (-CH₂CH₂CH₂OH), 2-hydroxypropyl (-CH₂CH(OH)CH₃), 1-hydroxypropyl (-CH(OH)CH₂CH₃), and the like. Hydroxyalkyl can be further substituted by an appropriate substituent.

The term "halogen" or "halo" means fluoro (-F), chloro (-CI), bromo (-Br), and iodine (-I).

The term "alkoxy" means alkyl that is bound to another group via an oxygen atom (that is, -O-alkyl). Examples of alkoxy include, but not limited to, methoxy (-O-methyl), ethoxy (-O-ethyl), propoxy (-O-propyl), -O-isopropyl, -O-2-methyl-1-propyl, -O-2-methyl-2-propyl, -O-2-methyl-1-butyl, -O-3-methyl-1-butyl, -O-2-methyl-3-butyl, -O-2,2-dimethyl-1-propyl, -0-2-methyl-1-pentyl, 3-O-methyl-1-pentyl, -O-4-methyl-1-pentyl, - O-2-methyl-2-pentyl, -O-3-methyl-2-pentyl, -O-4-methyl-2-pentyl, -O-2,2-dimethyl-1-butyl, -O-3,3-dimethyl-1-butyl, -O-2-ethyl-1-butyl, -O-butyl, -O-isobutyl, -O-t-butyl, -O-pentyl, - O-isopentyl, -O-neopentyl, and -O-hexyl. Alkoxy can be further substituted by an appropriate substituent.

The term "haloalkyl" means alkyl that is substituted by at least one halogen. Haloalkyl includes fluoroalkyl, chloroalkyl, bromoalkyl, and iodoalkyl. Examples of haloalkyl include, but not limited to, fluoromethyl, chloromethyl, bromomethyl, iodomethyl, fluoroethyl, chloroethyl, bromoethyl, iodoethyl, fluoropropyl, chloropropyl, bromopropyl, iodopropyl, fluorobutyl, chlorobutyl, bromobutyl, iodobutyl, fluoropentyl, chloropentyl, bromopentyl, iodopentyl, fluorohexyl, chlorohexyl, bromohexyl, iodohexyl, fluoroheptyl, chloroheptyl, bromoheptyl, iodoheptyl, fluorooctyl, chlorooctyl, bromooctyl, iodooctyl, and the like. Haloalkyl can be further substituted by an appropriate substituent.

The term "haloalkoxy" means alkoxy that is substituted by at least one halogen (that is, -O-haloalkyl). Haloalkoxy includes fluoroalkoxy, chloroalkoxy, bromoalkoxy, and iodoalkoxy.

The term "halohydroxyalkoxy" means haloalkoxy that is substituted by a hydroxy group. Halohydroxyalkoxy includes fluorohydroxyalkoxy, chlorohydroxyalkoxy, bromohydroxyalkoxy, and iodohydroxyalkoxy. Examples of halohydroxyalkoxy include -O-CH(F)(OH), -O-CH₂CH(F)(OH), - O-CH(OH)-CH₂(F), -O-CH₂-CH(F)(OH), -O-CH(OH)-CH₂-CH₂(F), - O-CH₂-CH(OH)-CH₂(F), -O-CH(CH₂-F)(CH₂OH), -O-CH₂-CH₂-CH(F)(OH), and the like.

The term "nitro" means -NO₂.

The term "amino" means -NH₂.

The term "aminoalkyl" means an alkyl group that is substituted by an amino group. Examples of aminoalkyl include, but not limited to, aminomethyl, aminoethyl, aminopropyl, aminoisopropyl, aminobutyl, aminopentyl, aminohexyl, and aminooctyl.

The term "substituent" means one or more atoms or an atomic group that is introduced in a certain chemical structural formula. Examples of substituents include, for example, C₁₋₈ alkyl (such as methyl, ethyl, n-propyl, iso-propyl, n-butyl, sec-butyl, iso-butyl, tert-butyl, or n-hexyl, or an isomer thereof), C₂₋₈ alkenyl (such as vinyl, allyl, -CH=CH(CH₃), -CH=C(CH₃)₂, -C(CH₃)=CH₂, -C(CH₃)=CH(CH₃), and - C(CH₂CH₃)=CH₂), C₂₋₈ alkynyl (such as ethynyl, -C≡CH(CH₃), - C≡C(CH₂CH₃), -CH₂C≡CH, -CH₂C≡C(CH₃), and - CH₂C≡C(CH₂CH₃)), alkoxy, hydroxy, halogen, haloalkyl, cycloalkyl (such as cyclopropyl, cyclobutyl, cyclopentyl, or cyclohexyl), amino, nitro, acyl (such as acetyl) (-COCH₃), carboxyl (-COOH), ester (-COOR^{x}, where R^{x} is C₁₋₆ alkyl or the like), amide (-CONR^{y}R^{z}, where R^{y} and R^{z} are each independently H, C₁₋₆ alkyl, or the like), thiol (-SH), sulfonic acid (-SO₃H), nitrile (-CN), aromatic rings (such as aryl, phenyl, benzoyl, or naphthalenyl), heterocyclic rings (such as pyrrolidinyl, tetrahydrofuranyl, pyrrolyl, furanyl, thiophenyl, piperidinyl, oxanyl, or pyridinyl), and the like.

The term "pharmaceutically acceptable salt" refers to a salt that is not harmful to mammals, particularly to humans. Pharmaceutically acceptable salts can be formed using nontoxic acids or bases, including inorganic acids or inorganic bases, or organic acids or organic bases. Examples of pharmaceutically acceptable salts include metal salts formed from aluminum, calcium, lithium, magnesium, potassium, sodium, zinc, and the like, and organic salts formed from lysine, N,N'-dibenzylethylenediamine, chloroprocaine, choline, diethanolamine, ethylenediamine, meglumine (N-methylglucamine), procaine and the like. Moreover, pharmaceutically acceptable salts encompass acid-addition salts and base-addition salts.

The term "pharmaceutically acceptable carrier" means pharmaceutically acceptable materials, compositions, or vehicles such as physiological saline solutions, liquid or solid fillers, diluents, solvents, or encapsulants. Examples of pharmaceutically acceptable carriers include water, saline water, physiological saline water or phosphate buffered saline water (PBS), sodium chloride injection solution, Ringer's injection solution, isotonic dextrose injection solution, sterile water injection solution, dextrose, lactated Ringer's injection solution, and the like.

The term "effective dose" refers to an amount of a compound or a composition which can bring about an intended effect. For example, in some embodiments, the effective dose refers to an amount of a compound or a composition which enables optical or radiological imaging of substances (such as α-synuclein aggregate) that accumulate in the brain.

The term "solvate" means a solvent-containing compound that is formed by association of one or more solvent molecules to a compound. Solvates encompass, for example, monosolvates, disolvates, trisolvates, and tetrasolvates. Moreover, solvates encompass hydrates.

The term "hydrate" means a compound further containing a stoichiometric or a non-stoichiometric amount of water constrained by non-covalent bonding intermolecular force, or a salt thereof. Hydrates encompass, for example, monohydrates, dihydrates, trihydrates, and tetrahydrates.

The term "treatment" means moderating or remitting progress, severity and/or duration of a disease or condition.

The term "prevention" means reducing a risk of catching or progressing a predetermined disease or condition, or reducing or inhibiting relapse, start or progress of one or more symptoms of a predetermined disease or condition.

### <α-synuclein aggregate binding agent>

The α-synuclein aggregate binding agent in accordance with an aspect of the present invention (hereinafter also referred to as "binding agent") contains a compound represented by the following formula (I), a pharmaceutically acceptable salt thereof, or a solvate thereof.

Among the compounds disclosed in Patent Literature 1, the compound represented by the following formula (I) is a compound having a buteninyl bond instead of a butadiene bond and having a benzothiazole structure or a benzofuran structure. The compound represented by the following formula (I) has been confirmed to have higher binding selectivity for the α-synuclein aggregate than to an aggregate of tau proteins or amyloid-β peptides. Similarly, the pharmaceutically acceptable salt of the compound represented by the following formula (I) and the solvate of the compound represented by the following formula (I) have higher binding selectivity for the α-synuclein aggregate than to an aggregate of tau proteins or amyloid-β peptides.

The compound represented by the following formula (I) also fluoresces. In addition, one or more atoms of the compound represented by the following formula (I) can be radioisotopes thereof.

Therefore, the binding agent in accordance with an aspect of the present invention can be used as a molecular probe for optical imaging or radiological imaging of the α-synuclein aggregate accumulated in the brain.

In addition, the compound represented by the following formula (I), the pharmaceutically acceptable salt thereof, and the solvate thereof have high binding selectivity for the α-synuclein aggregate as described above, and therefore can inhibit aggregation of α-synuclein (details will be described later).

Note that α-synuclein is a protein that localizes at a neuronal synapse also in the normal brain, and the α-synuclein aggregate is formed by α-synuclein assemblies. 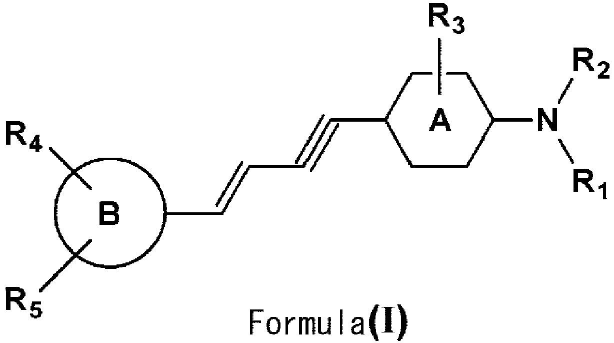 in the formula (I),
R₁ and R₂ are each independently selected from the group consisting of hydrogen, alkyl, alkenyl, acyl, and hydroxyalkyl;
R₃ is hydrogen or halogen;
the ring A is a benzene or pyridine ring;
the ring B is represented by the following formula (i) or (ii): 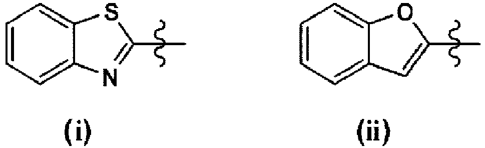
R₄ and R₅ are each independently selected from the group consisting of hydrogen, hydroxy, alkoxy, haloalkoxy, halohydroxyalkoxy, and aminoalkyl.

In one embodiment, the ring B is represented by the formula (i). In another embodiment, the ring B is represented by the formula (ii).

In a case where the ring B is represented by the formula (i), R₄ and R₅ can be at substitutable positions in the benzothiazole ring of the formula (i). Preferably, R₄ and R₅ are at position 6 and position 5 in the benzothiazole ring of the formula (i), respectively. In a case where the ring B is represented by the formula (ii), R₄ and R₅ can be at substitutable positions in the benzofuran ring of the formula (ii). Preferably, R₄ and R₅ are at position 5 and position 6 in the benzofuran ring of the formula (ii), respectively.

In one embodiment, the ring A is a pyridine ring. In another embodiment, the ring A is a benzene ring. Preferably, the ring A is the pyridine ring represented by the following structural formula, in the orientation of the structural formula of the formula (I).

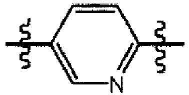

In one embodiment, R₁ and R₂ are both hydrogen.

In one embodiment, R₁ and R₂ are each independently hydrogen or alkyl, in particular C₁₋₈ alkyl, preferably methyl. In another embodiment, R₁ is hydrogen, R₂ is alkyl, in particular C₁₋₆ alkyl, preferably methyl. In yet another embodiment, R₁ and R₂ are both alkyl, preferably both C₁₋₆ alkyl, more preferably both methyl.

In one embodiment, R₁ and R₂ are each independently hydrogen or alkenyl, in particular C₂₋₈ alkenyl, preferably allyl (-CH₂CH=CH₂) or hepta-1,6-diene-4-yl (-CH₂-(CH₂CH=CH₂)₂). In another embodiment, R₁ is hydrogen, R₂ is alkenyl, in particular C₂₋₈ alkenyl, preferably allyl (-CH₂CH=CH₂) or hepta-1,6-diene-4-yl (-CH₂-(CH₂CH=CH₂)₂). In yet another embodiment, R₁ and R₂ are both alkenyl, preferably both C₂₋₈ alkenyl, more preferably both allyl (-CH₂CH=CH₂) or hepta-1,6-diene-4-yl (-CH₂-(CH₂CH=CH₂)₂).

In one embodiment, R₁ and R₂ are each independently hydrogen or acyl, in particular C₁₋₈ acyl, preferably acetyl (-COCH₃). In another embodiment, R₁ is hydrogen, R₂ is acyl, in particular C₁₋₈ acyl, and preferably acetyl (-COCH₃). In yet another embodiment, R₁ and R₂ are both acyl, preferably both C₁₋₈ acyl, more preferably acetyl (-COCH₃).

In one embodiment, R₁ and R₂ are each independently hydrogen or hydroxyalkyl, in particular hydroxy C₁₋₈ alkyl, preferably hydroxypropyl, more preferably 3-hydroxypropyl (-CH₂CH₂CH₂OH). In another embodiment, R₁ is hydrogen, R₂ is hydroxyalkyl, in particular hydroxy C₁₋₈ alkyl, preferably hydroxypropyl, more preferably 3-hydroxypropyl (-CH₂CH₂CH₂OH). In yet another embodiment, R₁ and R₂ are both hydroxyalkyl, preferably both hydroxy C₁₋₈ alkyl, more preferably hydroxypropyl, further preferably 3-hydroxypropyl (-CH₂CH₂CH₂OH) .

In one embodiment, R₃ is hydrogen. In another embodiment, R₃ is halogen, that is, F, Cl, Br or I. Preferably, R₃ is F. Preferably, R₃ is ¹⁸F. In the orientation of the structural formula of the formula (I), R₃ is preferably at the following position.

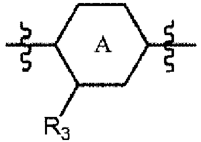

In the orientation of the structural formula of the formula (I), the ring A and R₃ preferably have the following relationship.

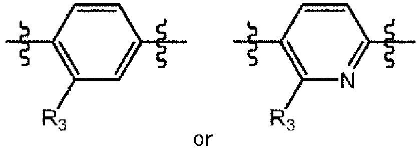

In one embodiment, R₄ and R₅ are both hydrogen.

In one embodiment, R₄ and R₅ are each independently hydrogen or hydroxy. In another embodiment, R₄ is hydroxy, and R₅ is hydrogen. In yet another embodiment, R₄ is hydrogen, and R₅ is hydroxy. In yet another embodiment, R₄ and R₅ are both hydroxy.

In one embodiment, R₄ and R₅ are each independently hydrogen or alkoxy, in particular methoxy. In another embodiment, R₄ is alkoxy, in particular methoxy, and R₅ is hydrogen. In yet another embodiment, R₄ is hydrogen, R₅ is alkoxy, in particular methoxy. In yet another embodiment, R₄ and R₅ are both alkoxy, preferably both methoxy.

In one embodiment, R₄ and R₅ are each independently hydrogen or haloalkoxy, in particular fluoroalkoxy, chloroalkoxy, bromoalkoxy, or iodoalkoxy. In another embodiment, R₄ is haloalkoxy, in particular fluoroalkoxy, chloroalkoxy, bromoalkoxy, or iodoalkoxy, and R₅ is hydrogen. In yet another embodiment, R₄ is hydrogen, and R₅ is haloalkoxy, in particular fluoroalkoxy, chloroalkoxy, bromoalkoxy, or iodoalkoxy. In yet another embodiment, R₄ and R₅ are both haloalkoxy, in particular both fluoroalkoxy, chloroalkoxy, bromoalkoxy, or iodoalkoxy. In one embodiment, fluoroalkoxy contains a radioisotope.

In one embodiment, R₄ and R₅ are each independently hydrogen or halohydroxyalkoxy, in particular fluorohydroxyalkoxy, preferably fluorohydroxy C₁₋₃ alkoxy, more preferably -O-CH₂-CH(OH)-CH₂(F) or -O-CH(CH₂-F)(CH₂OH). In another embodiment, R₄ is halohydroxyalkoxy, in particular fluorohydroxyalkoxy, preferably fluorohydroxy C₁₋₃ alkoxy, more preferably -O-CH₂-CH(OH)-CH₂(F) or -O-CH(CH₂-F)(CH₂OH), and R₅ is hydrogen. In yet another embodiment, R₄ is hydrogen, R₅ is halohydroxyalkoxy, in particular fluorohydroxyalkoxy, preferably fluorohydroxy C₁₋₃ alkoxy, more preferably -O-CH₂-CH(OH)-CH₂(F) or -O-CH(CH₂-F)(CH₂OH). In yet another embodiment, R₄ and R₅ are both halohydroxyalkoxy, preferably both fluorohydroxyalkoxy, more preferably both fluorohydroxy C₁₋₃ alkoxy, further preferably both -O-CH₂-CH(OH)-CH₂(F) or -O-CH(CH₂-F)(CH₂OH). In one embodiment, fluorohydroxyalkoxy contains a radioisotope. Preferably, such fluorohydroxyalkoxy is -O-CH₂-CH(OH)-CH₂(¹⁸F) or -O-CH(CH₂-¹⁸F)(CH₂OH).

In one embodiment, R₄ and R₅ are each independently hydrogen or aminoalkyl, in particular aminomethyl or aminoethyl. In another embodiment, R₄ is aminoalkyl, in particular aminomethyl or aminoethyl, and R₅ is hydrogen. In yet another embodiment, R₄ is hydrogen, and R₅ is aminoalkyl, in particular aminomethyl or aminoethyl. In yet another embodiment, R₄ and R₅ are both aminoalkyl, preferably both aminomethyl or aminoethyl.

In one embodiment, the compound represented by the formula (I) is a compound represented by the following formula (II): 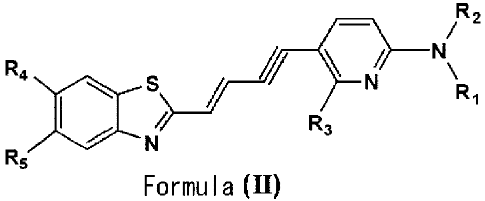 wherein, R₁, R₂, R₃, R₄, and R₅ are similar to R₁, R₂, R₃, R₄, and R₅, respectively, in the formula (I).

In one embodiment, the compound represented by the formula (I) is a compound represented by the following formula (III): 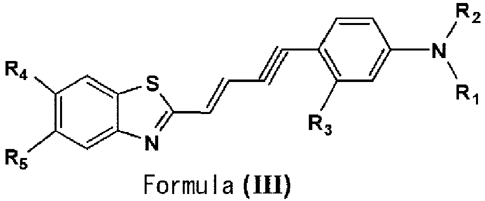 wherein, R₁, R₂, R₃, R₄, and R₅ are similar to R₁, R₂, R₃, R₄, and R₅, respectively, in the formula (I).

In one embodiment, the compound represented by the formula (I) is a compound represented by the following formula (IV): 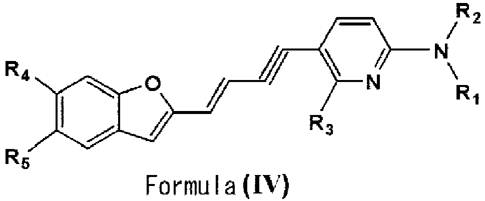 wherein, R₁, R₂, R₃, R₄, and R₅ are similar to R₁, R₂, R₃, R₄, and R₅, respectively, in the formula (I).

In one embodiment, the compound represented by the formula (I) is a compound represented by the following formula (V): 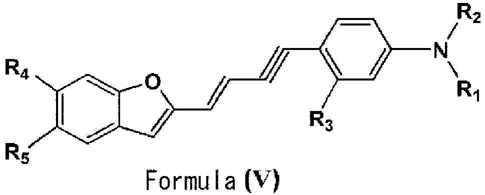 wherein, R₁, R₂, R₃, R₄, and R₅ are similar to R₁, R₂, R₃, R₄, and R₅, respectively, in the formula (I).

In one embodiment, in the compounds of the formulae (I) through (V), one or more atoms are radioisotopes thereof. The radioisotope is selected from the group consisting of ¹⁵O, ¹³N, ¹¹C, ¹⁸F and the like, but is not particularly limited. Preferably, the radioisotope is ¹¹C or ¹⁹F. Among these, considering that a half-life of ¹¹C is approximately 20 minutes and a half-life of ¹⁸F is approximately 110 minutes, a compound that is labeled with ¹⁸F would be commercially more useful. Therefore, the radioisotope is most preferably ¹⁸F.

Preferably, one or more of R₁ through R₅ are groups each of which contains a radioisotope. Further preferably, one or both of R₁ and R₂ are groups each of which contains a radioisotope, for example, groups each of which contains ¹¹C (such as [¹¹C]alkyl including ¹¹CH₃). More preferably, R₃ is a group containing a radioisotope, for example, -¹⁸F. More preferably, one or both of R₄ and R₅ are groups each of which contains a radioisotope, for example, groups each of which contains ¹¹C (such as [¹¹C]alkoxy including -O¹¹CH₃), or groups each of which contains ¹⁸F (such as [¹⁸F]fluorohydroxyalkoxy including -O-CH₂-CH(OH)-CH₂(¹⁸F) and -O-CH(CH₂-¹⁸F)(CH₂OH)). Here, [¹¹C]alkyl indicates that one or more carbon atoms in carbon atoms constituting alkyl are ¹¹C. [¹¹C]alkoxy indicates that one or more carbon atoms in carbon atoms constituting alkoxy are ¹¹C. [¹⁸F]fluorohydroxyalkoxy means a group in which ¹⁸F is bound to hydroxyalkoxy.

Specific examples of the compound represented by the formula (I) include the following compounds:

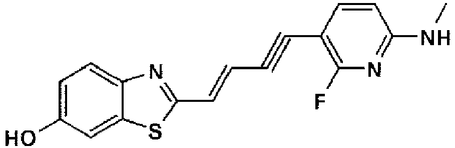

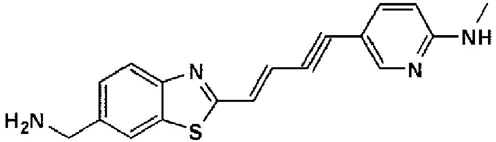

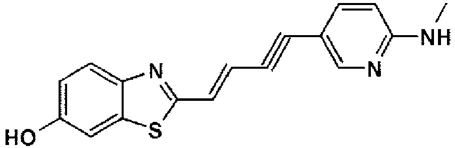

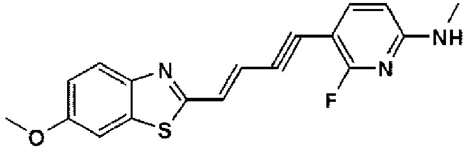

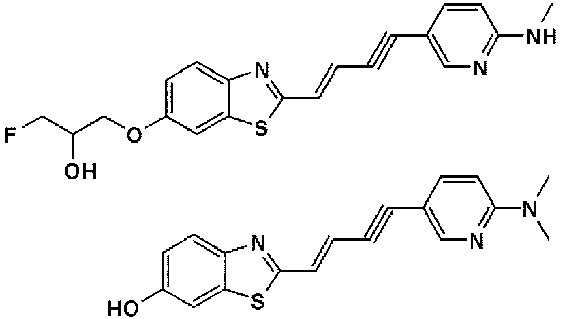

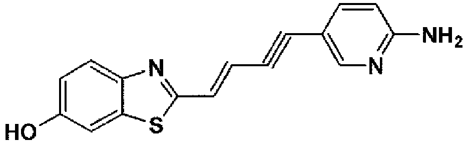

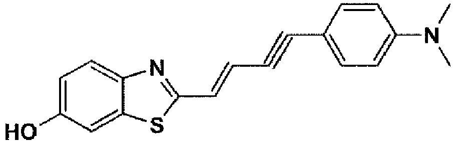

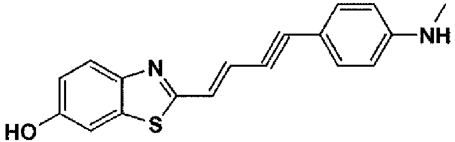

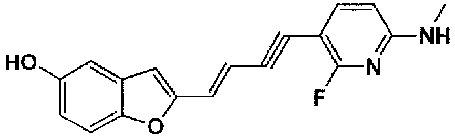

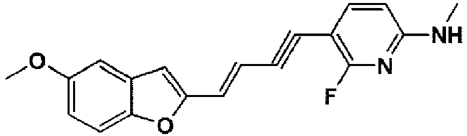

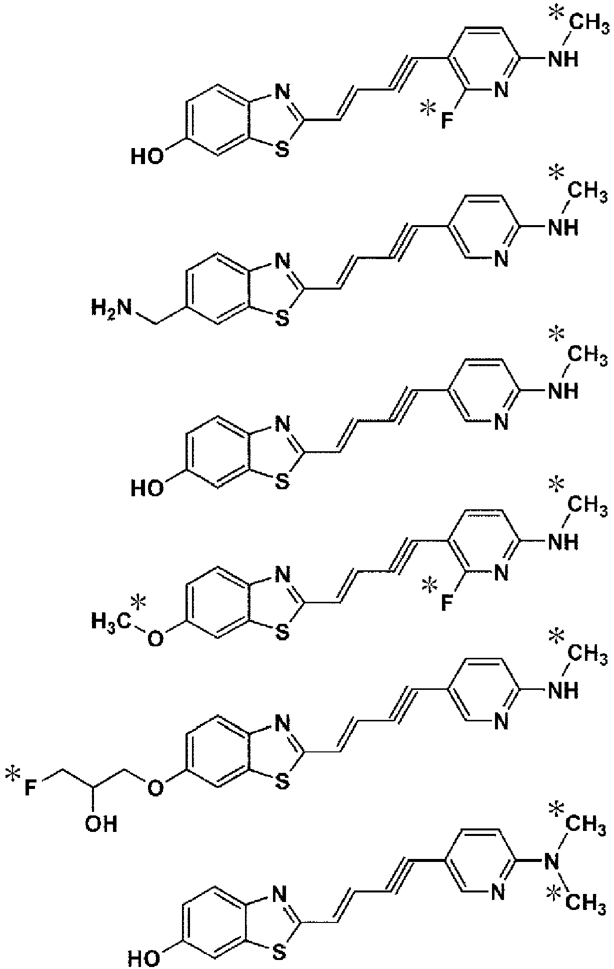

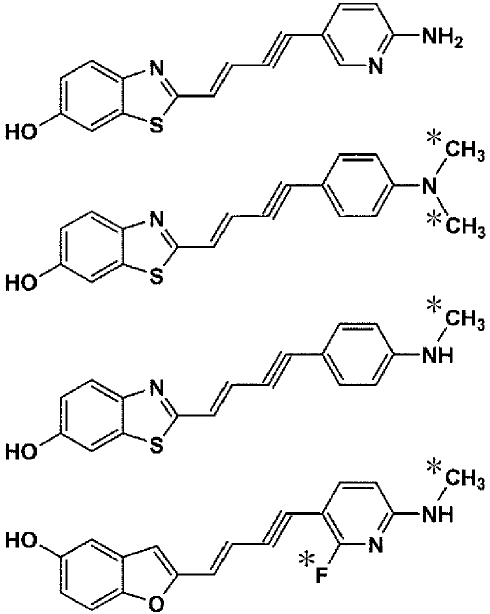

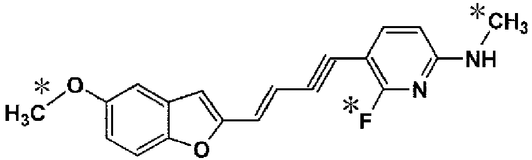

In one embodiment, in the specific compounds above, one or more atoms are radioisotopes thereof. Preferably, a carbon atom bound to a nitrogen atom of an amino group in aminobenzil or aminopyridinyl bound to a benzene or pyridine ring is a radioisotope ¹¹C. Preferably, F in the above specific compounds is a radioisotope ¹⁸F. Preferably, a carbon atom of a methoxy group bound to a benzothiazole ring is a radioisotope ¹¹C. Further preferably, an atom with the symbol * in the structural formulae of the above specific compounds (where there are two symbols * in a structural formula, one or two of them) is a radioisotope thereof, for example, ¹¹C or ¹⁸F. Herein, names such as [¹¹C]C05-04 mean that ¹¹C is above the atom with the symbol * in the structural formula of C05-04, and the like.

Although there is no particular limitation on the method for synthesizing the compound represented by the formula (I), the compound represented by the formula (I) can be produced, for example, with reference to the synthesis method disclosed in Patent Literature 1.

Note that, among the compounds represented by the formula (I), a compound represented by the following formula, a pharmaceutically acceptable salt thereof, or a solvate thereof is a novel compound. The compounds represented by the following formulae are useful as the α-synuclein aggregate binding agent, but can also be used for applications other than the employment as the α-synuclein aggregate binding agent. 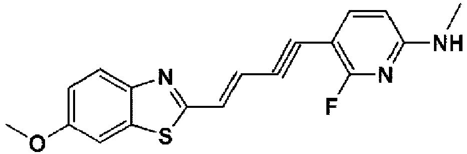 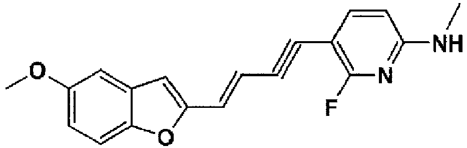 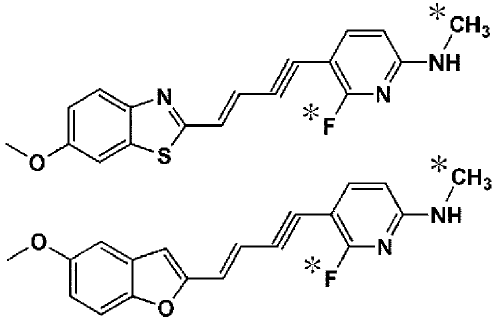 wherein at least one of atoms with the symbol * is a radioisotope thereof.

Those novel compounds can be synthesized, for example, by synthesis methods shown in Examples described later from intermediates below, respectively.

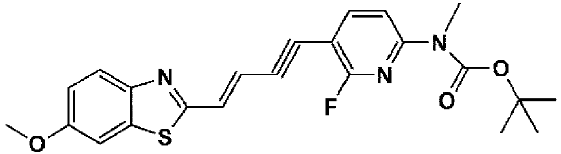

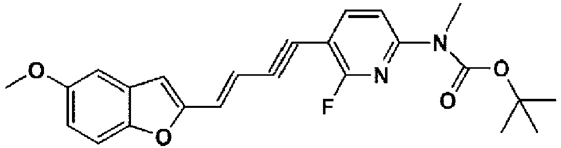

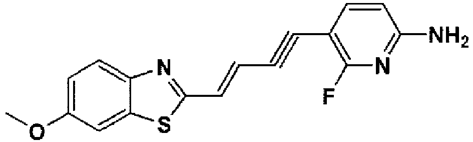

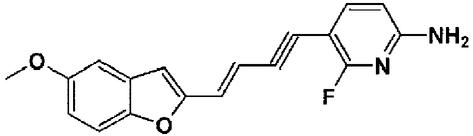

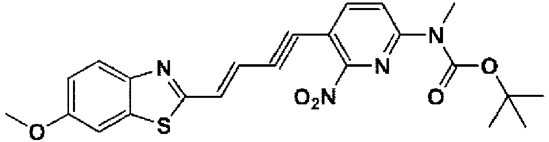

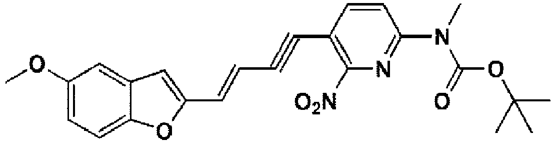

The α-synuclein aggregate binding agent can contain a pharmaceutically acceptable carrier. Examples of the pharmaceutically acceptable carrier include water, saline water, physiological saline water or phosphate buffered saline water (PBS), sodium chloride injection solution, Ringer's injection solution, isotonic dextrose injection solution, sterile water injection solution, dextrose, lactated Ringer's injection solution and the like.

Contents of the compound represented by the formula (I), a pharmaceutically acceptable salt thereof, or a solvate thereof, and a pharmaceutically acceptable carrier, which are contained in the α-synuclein aggregate binding agent, are not particularly limited, and are determined based on various factors such as: the type of the compound that is used: the age, weight, health conditions, sex, and content of diet of a mammal to which the substances are administered; the number of times of administration and the route of administration; the period of treatment; other drugs that are concurrently used, and the like. A content of the pharmaceutically acceptable carrier can be 1% by weight to 99% by weight of the α-synuclein aggregate binding agent. The α-synuclein aggregate binding agent is prepared so that the compound represented by the formula (I) can be administered, for example, in an amount of 5 ng/kg to 5 mg/kg, preferably, a lower limit of 5 ng/kg or more, 0.01 mg/kg or more, 0.05 mg/kg or more, or 0. 1 mg/kg or more, an upper limit of 5 mg/kg or less, 3 mg/kg or less, 1 mg/kg or less, or 20 µg/kg or less, in terms of amount of the compound represented by the formula (I) per weight (kg) of a subject.

### <Composition for optical imaging of α-synuclein aggregate>

The composition for optical imaging of an α-synuclein aggregate in accordance with an aspect of the present invention (hereinafter also referred to as "composition for optical imaging") contains the binding agent in accordance with an aspect of the present invention. The optical imaging encompasses in vitro, ex vivo, and in vivo imaging.

Examples of the optical imaging include fluorescence microscope measurement and multiphoton, two-photon, and near infrared fluorescence imaging.

The composition for optical imaging can contain the pharmaceutically acceptable carrier. Examples of the pharmaceutically acceptable carrier include water, saline water, physiological saline water or phosphate buffered saline water (PBS), sodium chloride injection solution, Ringer's injection solution, isotonic dextrose injection solution, sterile water injection solution, dextrose, lactated Ringer's injection solution and the like.

Contents of the compound represented by the formula (I), a pharmaceutically acceptable salt thereof, or a solvate thereof, and a pharmaceutically acceptable carrier, which are contained in the composition for optical imaging, are not particularly limited, and are determined based on various factors such as: the type of the compound that is used: the age, weight, health conditions, sex, and content of diet of a mammal to which the substances are administered; the number of times of administration and the route of administration; the period of treatment; other drugs that are concurrently used, and the like. A content of the pharmaceutically acceptable carrier can be 1% by weight to 99% by weight of the composition for optical imaging. The composition for optical imaging is prepared so that the compound represented by the formula (I) can be administered, for example, in an amount of 0.01 mg/kg to 5 mg/kg, preferably 0.05 mg/kg to 3 mg/kg, further preferably 0.1 mg/kg to 1 mg/kg, in terms of amount (mg) of the compound represented by the formula (I) per weight (kg) of a subject.

### <Composition for radiological imaging of α-synuclein aggregate>

The composition for radiological imaging of an α-synuclein aggregate in accordance with an aspect of the present invention (hereinafter also referred to as "composition for radiological imaging") contains the binding agent in accordance with an aspect of the present invention. The radiological imaging encompasses in vitro, ex vivo, and in vivo imaging.

Examples of the radiological imaging include positron emission tomography (PET), single-photon emission computed tomography (SPECT), and autoradiography.

The composition for radiological imaging can contain the pharmaceutically acceptable carrier. Examples of the pharmaceutically acceptable carrier include water, saline water, physiological saline water or phosphate buffered saline water (PBS), sodium chloride injection solution, Ringer's injection solution, isotonic dextrose injection solution, sterile water injection solution, dextrose, lactated Ringer's injection solution and the like.

Contents of the compound represented by the formula (I), a pharmaceutically acceptable salt thereof, or a solvate thereof, and a pharmaceutically acceptable carrier, which are contained in the composition for radiological imaging, are not particularly limited, and are determined based on various factors such as: the type of the compound that is used: the age, weight, health conditions, sex, and content of diet of a mammal to which the substances are administered; the number of times of administration and the route of administration; the period of treatment; other drugs that are concurrently used, and the like. A content of the pharmaceutically acceptable carrier can be 1% by weight to 99% by weight of the composition for radiological imaging. The composition for radiological imaging is prepared so that the compound represented by the formula (I) can be administered, for example, in an amount of 5 ng/kg to 5 mg/kg, preferably 5 ng to 20 µg/kg, in terms of amount of the compound represented by the formula (I) per weight (kg) of a subject.

### <Diagnostic agent for disease associated with α-synuclein aggregate or companion diagnostic agent for treating or preventing that disease>

The diagnostic agent for a disease associated with an α-synuclein aggregate or the companion diagnostic agent for treating or preventing the disease in accordance with an aspect of the present invention (hereinafter also referred to as "companion diagnostic agent") contains the binding agent in accordance with an aspect of the present invention. The companion diagnostic agent for treatment is a diagnostic agent for determining when the disease is found, whether the disease would be treated or not. The companion diagnostic agent for prevention is a diagnostic agent for, when a precursor state of the diseases is found, predicting a future onset or determining whether prevention is expected to control the onset.

By checking data pertaining to the quantity and/or distribution of the α-synuclein aggregate in the brain obtained from a subject with use of the diagnostic agent against a previously obtained correlation between the disease and the quantity and/or distribution of the α-synuclein aggregate, it is possible to diagnose the disease of the subject (specifically, the presence or absence of affection, severity, seizure likelihood, and the like of the disease).

Moreover, by checking data pertaining to the quantity and/or distribution of the α-synuclein aggregate in the brain obtained from a subject with use of the companion diagnostic agent against a previously obtained correlation between the disease and the quantity and/or distribution of the α-synuclein aggregate in the brain, the disease condition of the subject is grasped, and it is possible to lay out a plan for prevention/treatment of the disease (the type and combination, dosage, usage, and the like of preventive/therapeutic agents to be administered) based on that disease condition.

One embodiment of the present invention relates to a medicine for treatment or prevention of a disease associated with an α-synuclein aggregate, wherein the medicine is administered in a dosage regimen based on data pertaining to a quantity and/or distribution of an α-synuclein aggregate in the brain obtained in the companion diagnosis.

### <Diagnostic kit for disease associated with substance accumulated in brain>

The diagnostic kit for a disease associated with a substance accumulated in the brain in accordance with an aspect of the present invention (hereinafter also referred to as "diagnostic kit") includes: the binding agent in accordance with an aspect of the present invention; and at least one compound selected from 2-((1E,3E)-4-(6-(methylamino)pyridin-3-yl)buta-1,3-dienyl)benzo[d]thiazol-6-ol, 2-((1E,3E)-4-(6-((11)C-methylamino)pyridin-3-yl)buta-1,3-dienyl)benzo[d]thiazol-6-ol, 1-fluoro-3-(2-((1E,3E)-4-(6-(methylamino)pyridin-3-yl)buta-1,3-dienyl)benzo[d]thiazol-6-yloxy)propan-2-ol, and 1-(18)F-fluoro-3-(2-((1E,3E)-4-(6-(methylamino)pyridin-3-yl)buta-1,3-dienyl) benzo [d]thiazol-6-yloxy)propan-2-ol.

The substance accumulated in the brain includes an α-synuclein aggregate, and aggregates of tau proteins and amyloid-β peptides.

The disease associated with a substance accumulated in the brain includes: Parkinson's disease, dementia with Lewy bodies (DLB), and multiple system atrophy (MSA) which are diseases associated with an α-synuclein aggregate; and Alzheimer's disease (AD) and frontotemporal lobar degeneration which are diseases associated with an aggregate of tau proteins or amyloid-β peptides.

It has been found that the binding agent in accordance with an aspect of the present invention has higher binding selectivity for an α-synuclein aggregate than to an aggregate of tau proteins or amyloid-β peptides, whereas 2-((1E,3E)-4-(6-(methylamino)pyridin-3-yl)buta-1,3-dienyl)benzo[d]thiazol-6-ol, 2-((1E,3E)-4-(6-((11)C-methylamino)pyridin-3-yl)buta-1,3-dienyl)benzo[d]thiazol-6-ol, 1-fluoro-3-(2-((1E,3E)-4-(6-(methylamino)pyridin-3-yl)buta-1,3-dienyl)benzo[d]thiazol-6-yloxy)propan-2-ol, and 1-(18)F-fluoro-3-(2-((1E,3E)-4-(6-(methylamino)pyridin-3-yl)buta-1,3-dienyl)benzo[d]thiazol-6-yloxy)propan-2-ol has higher binding selectivity for an aggregate of tau proteins than to an α-synuclein aggregate.

The diagnostic kit in accordance with an aspect of the present invention includes both the former having the high binding selectivity for the α-synuclein aggregate and the latter having the high binding selectivity for the aggregate of tau proteins. Therefore, by comparing a detection result of the former (the quantity and/or distribution of light or radioactivity which has been detected) with a detection result of the latter, it is possible to classify which substance (specifically, the α-synuclein or tau protein aggregate) is present in each of imaged areas, and it is also possible to quantify the amount of each substance present. Thus, the disease associated with an α-synuclein aggregate and/or the disease associated with a tau protein aggregate can be diagnosed with high accuracy. For example, (i) by specifying, in advance, the ratio between binding performance to the α-synuclein aggregate and binding performance to the tau protein aggregate for the binding agent in accordance with an aspect of the present invention and for each of 2-((1E,3E)-4-(6-(methylamino)pyridin-3-yl)buta-1,3-dienyl)benzo[d]thiazol-6-ol, 2-((1E,3E)-4-(6-((11)C-methylamino)pyridin-3-yl)buta-1,3-dienyl)benzo[d]thiazol-6-ol, and the like and (ii) then by measuring the amount ratio of light or radioactivity in each area in the brain after the former and the latter are administered to the subject, it is possible to classify whether the α-synuclein or tau protein aggregate is present in that area and to quantify the amount of each substance, based on the relation between the ratio specified in advance and the measured amount ratio.

It is also possible to make the diagnostic kit in accordance with an aspect of the present invention a diagnostic kit combined with an imaging agent for amyloid-β. The diagnostic kit in which the diagnostic kit in accordance with an aspect of the present invention is combined with the imaging agent for amyloid-β makes it possible to classify which substance (specifically, the α-synuclein, tau protein, or amyloid-β aggregate) is present in each of imaged areas, and also makes it possible to quantify the amount of each substance present. Thus, the diseases associated with α-synuclein, tau proteins, and/or amyloid-β peptides can be diagnosed with high accuracy.

One embodiment of the present invention relates to a medicine for treatment or prevention of a disease associated with a substance accumulated in the brain, in which the medicine is administered in a dosage regimen based on data pertaining to a quantity and/or distribution of a substance accumulated in the brain obtained with the diagnostic kit.

### <Optical imaging method>

The optical imaging method in accordance with an aspect of the present invention includes the step of externally irradiating the brain of a living subject, to which the binding agent in accordance with an aspect of the present invention has been administered, with light having the first wavelength, and then detecting light which is emitted from the brain and has the second wavelength different from the first wavelength.

In a case where an effective dose of the binding agent is administered to a subject, the binding agent that has been delivered to the living brain binds to an α-synuclein aggregate present in the living brain. By (i) externally irradiating the brain of the living subject, to which the binding agent has been administered, with light having the first wavelength for exciting the binding agent, and (ii) detecting light (e.g., fluorescence) having the second wavelength emitted from the binding agent in the brain, it is possible to carry out optical imaging of the α-synuclein aggregate.

The subjects can be mammals. The mammals include, for example, a human, a rat, a mouse, a rabbit, a guinea pig, a hamster, a monkey, a dog, a ferret, and a minipig.

An administration method is not particularly limited and can be, for example, oral administration, or parenteral administration such as intravenous and intraperitoneal administrations. Preferably, intravenous or intraperitoneal administration is employed. Most preferably, intravenous administration is employed. The dosage is preferably 0.01 mg/kg to 5 mg/kg, 0.05 mg/kg to 3 mg/kg, or 0.1 mg/kg to 1 mg/kg, and most preferably 0.1 mg/kg to 1 mg/kg.

### <Radiological imaging method>

The radiological imaging method in accordance with an aspect of the present invention includes the step of detecting radioactivity emitted from the brain of a living subject to which the binding agent has been administered, which contains the compound represented by the formula (I) in which one or more atoms are radioisotopes thereof, a pharmaceutically acceptable salt thereof, or a solvate thereof.

In a case where an effective dose of the binding agent is administered to a subject, the binding agent that has been delivered to the living brain binds to an α-synuclein aggregate present in the living brain. The radiological imaging of the α-synuclein aggregate can be achieved by detecting the radioactivity emitted from the binding agent in the brain.

The subjects can be mammals. The mammals include, for example, a human, a rat, a mouse, a rabbit, a guinea pig, a hamster, a monkey, a dog, a ferret, and a minipig. Preferably, the mammal is a human.

An administration method is not particularly limited and can be, for example, oral administration, or parenteral administration such as intravenous and intraperitoneal administrations. Preferably, intravenous or intraperitoneal administration is employed. Most preferably, intravenous administration is employed. The dosage is preferably 5 ng/kg to 5 mg/kg, more preferably 5 ng to 20 µg/kg. The applied radiation dose is preferably 37 MBq to 7.4 GBq per individual, more preferably 370 GBq to 3700 GBq.

### <Method of screening for therapeutic or preventive agent for disease associated with α-synuclein aggregate in brain>

The method of screening for a therapeutic or preventive agent for a disease associated with an α-synuclein aggregate in the brain in accordance with an aspect of the present invention (hereinafter also referred to as screening method) includes the step of selecting a candidate substance based on a difference, which has been caused by administration of the candidate substance to the subject, in quantity and/or distribution of light or radioactivity which are detected in the foregoing <Optical imaging method> or <Radiological imaging method>.

Diseases associated with an α-synuclein aggregate in the brain are similar to those described above in <Diagnostic kit for disease associated with substance accumulated in brain>.

The subject and the administration method are similar to those described above in <Optical imaging method> and <Radiological imaging method>.

For example, in a case where, after administration of a candidate substance, the amount (intensity) of light (such as fluorescence of the binding agent) or radioactivity is reduced as compared with that prior to administration of the candidate substance, the candidate substance can be useful as a therapeutic compound for the disease or symptom.

Moreover, in a case where the quantity and/or distribution of light or radioactivity detected in a subject is compared with that of another mammal which is normal, and the quantity and/or distribution approaches, after administration of the candidate compound, that of the normal mammal as compared with the state prior to administration, the candidate compound can be useful as a therapeutic compound for the disease or symptom.

### <Method for carrying out quantification or determination of accumulation of α-synuclein aggregate in brain>

The method for carrying out quantification or determination of accumulation of an α-synuclein aggregate in the brain in accordance with an aspect of the present invention includes a step of externally irradiating the brain of a living subject, to which the binding agent has been administered, which contains the compound represented by the formula (I), a pharmaceutically acceptable salt thereof, or a solvate thereof, with light having the first wavelength, and then detecting light which is emitted from the brain and has the second wavelength different from the first wavelength, and quantifies or determines accumulation of the α-synuclein aggregate in the brain based on the quantity and/or distribution of the detected light. This method is a method for carrying out quantification or determination of accumulation of an α-synuclein aggregate in the brain by optical imaging.

The subject and the administration method are similar to those described above in <Optical imaging method>.

By determining differences between the quantity and/or distribution of light detected in the subject and those of another mammal which is normal, it is possible to quantify accumulation of the α-synuclein aggregate in the brain and to determine the presence or absence of accumulation of the α-synuclein aggregate in the brain.

In another embodiment, the method for carrying out quantification or determination of accumulation of an α-synuclein aggregate in the brain in accordance with an aspect of the present invention includes a step of detecting radioactivity emitted from the brain of a living subject, to which the binding agent has been administered, which contains the compound represented by the formula (I) in which one or more atoms are radioisotopes thereof, a pharmaceutically acceptable salt thereof, or a solvate thereof, and quantifies or determines accumulation of the α-synuclein aggregate in the brain based on the quantity and/or distribution of the detected radioactivity. This method is a method for carrying out quantification or determination of accumulation of an α-synuclein aggregate in the brain by radiological imaging.

The subject and the administration method are similar to those described above in <Radiological imaging method>.

By determining differences between the quantity and/or distribution of light or radioactivity detected in the subject and those of another mammal which is normal, it is possible to quantify accumulation of the α-synuclein aggregate in the brain and to determine the presence or absence of accumulation of the α-synuclein aggregate in the brain.

### <Method for carrying out determination of classification and detection of substance accumulated in brain>

The method for classification and detection of a substance accumulated in the brain in accordance with an aspect of the present invention includes: the first step of externally irradiating the brain of a living subject, to which the binding agent has been administered, which contains the compound represented by the formula (I), a pharmaceutically acceptable salt thereof, or a solvate thereof, with light having the first wavelength, and then detecting light which is emitted from the brain and has the second wavelength different from the first wavelength; and the second step of externally irradiating the brain of the living subject, to which at least one compound selected from 2-((1E,3E)-4-(6-(methylamino)pyridin-3-yl)buta-1,3-dienyl)benzo[d]thiazol-6-ol and 1-fluoro-3-(2-((1E,3E)-4-(6-(methylamino)pyridin-3-yl)buta-1,3-dienyl)benzo[d]thiazol-6-yloxy)propan-2-ol has been administered at a time point which is different from that of the first step, with light having the third wavelength, and then detecting light that is emitted from the brain and has the fourth wavelength different from the third wavelength, the determination being carried out based on data of a quantity and/or distribution of the light detected in the first step and on data of a quantity and/or distribution of the light detected in the second step. This method is a method for classification and detection of a substance accumulated in the brain by optical imaging.

The subject and the administration method are similar to those described above in <Optical imaging method>.

The method includes both the first step of detecting light emitted from the brain of the subject to which the binding agent having the high binding selectivity for an α-synuclein aggregate has been administered and the second step of detecting light emitted from the brain of the subject to which the substance having the high binding selectivity for a tau protein aggregate has been administered. Therefore, by comparing the detection result of the first step (the quantity and/or distribution of detected light) with the detection result of the second step, it is possible to determine classification as to whether the substance accumulated in the brain is an α-synuclein aggregate and/or a tau protein aggregate, or determine such accumulation.

In another embodiment, the method for classification and detection of a substance accumulated in the brain in accordance with an aspect of the present invention includes: the first step of detecting radioactivity that is emitted from the brain of a living subject to which the binding agent has been administered, which contains the compound represented by the formula (I) in which one or more atoms are radioisotopes thereof, a pharmaceutically acceptable salt thereof, or a solvate thereof; and the second step of detecting radioactivity that is emitted from the brain of the subject to which at least one compound selected from 2-((1E,3E)-4-(6-((11)C-methylamino)pyridin-3-yl)buta-1,3-dienyl)benzo[d]thiazol-6-ol and 1-(18)F-fluoro-3-(2-((lE,3E)-4-(6-(methylamino)pyridin-3-yl)buta- 1,3-dienyl)benzo[d]thiazol-6-yloxy)propan-2-ol has been administered at a time point which is different from that of the first step, the determination being carried out based on data of a quantity and/or distribution of the radioactivity detected in the first step and on data of a quantity and/or distribution of the radioactivity detected in the second step. This method is a method for classification and detection of a substance accumulated in the brain by radiological imaging.

The subject and the administration method are similar to those described above in <Radiological imaging method>.

The method includes both the first step of detecting radioactivity emitted from the brain of the subject to which the binding agent having the high binding selectivity for an α-synuclein aggregate has been administered and the second step of detecting radioactivity emitted from the brain of the subject to which the substance having the high binding selectivity for a tau protein aggregate has been administered. Therefore, by comparing the detection result of the first step (the quantity and/or distribution of detected radioactivity) with the detection result of the second step, it is possible to determine classification as to whether the substance accumulated in the brain is an α-synuclein aggregate and/or a tau protein aggregate, or determine such accumulation.

### <α-synuclein aggregation inhibitor>

The α-synuclein aggregation inhibitor in accordance with an aspect of the present invention (hereinafter also referred to as "aggregation inhibitor") contains the compound represented by the formula (I), a pharmaceutically acceptable salt thereof, or a solvate thereof. The compound represented by the formula (I), the pharmaceutically acceptable salt thereof, and the solvate thereof each of which is to be contained in the α-synuclein aggregation inhibitor in accordance with an aspect of the present invention are similar to the compound represented by the formula (I), the pharmaceutically acceptable salt thereof, and the solvate thereof in the above <α-synuclein aggregate binding agent>.

The α-synuclein aggregation inhibitor in accordance with an aspect of the present invention is capable of inhibiting the formation of an α-synuclein aggregate, as shown in Examples described later. Therefore, in a case where the α-synuclein aggregation inhibitor in accordance with an aspect of the present invention is administered to a mammal, the aggregation inhibitor that has been delivered to the living brain inhibits aggregation of the α-synuclein present in the living brain, and this makes it possible to prevent or treat diseases derived from the α-synuclein aggregate in the mammal, e.g., Parkinson's disease, dementia with Lewy bodies (DLB), multiple system atrophy (MSA), and the like, which are derived from the α-synuclein aggregate.

The compound represented by the formula (I), the pharmaceutically acceptable salt thereof, and the solvate thereof have high binding selectivity for the α-synuclein aggregate as described above.

It is inferred that the compound represented by the formula (I), the pharmaceutically acceptable salt thereof, and the solvate thereof have high binding selectivity for the α-synuclein aggregate and can control or inhibit the progression of the α-synuclein aggregation by binding to the α-synuclein aggregate when the α-synuclein aggregates.

The mammals to which the aggregation inhibitor is to be administered include, for example, a human, a rat, a mouse, a rabbit, a guinea pig, a hamster, a monkey, a dog, a ferret, and a minipig.

An administration method of the aggregation inhibitor is not particularly limited and can be, for example, oral administration, or parenteral administration such as intravenous and intraperitoneal administrations. It is possible to employ direct administration to the part (brain) where the α-synuclein aggregate is present. Preferably, intravenous or intraperitoneal administration is employed. Most preferably, intravenous administration is employed. The dosage is preferably 0.01 mg/kg to 5 mg/kg, 0.05 mg/kg to 3 mg/kg, or 0.1 mg/kg to 1 mg/kg, and most preferably 0.1 mg/kg to 1 mg/kg.

### Examples

The following description will discuss the present invention in further detail with reference to Examples. Note, however, that the present invention is not limited to those Examples.

The following compounds were used.

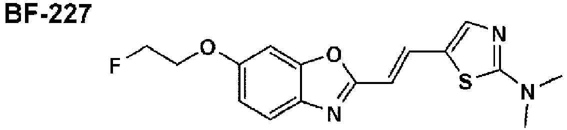

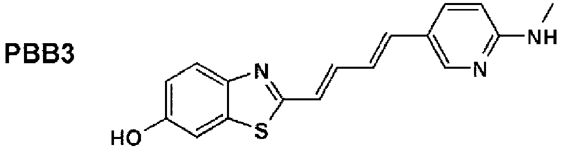

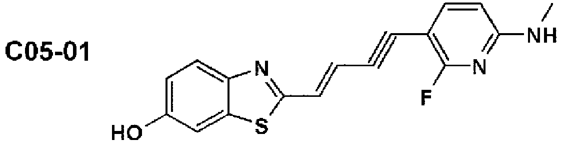

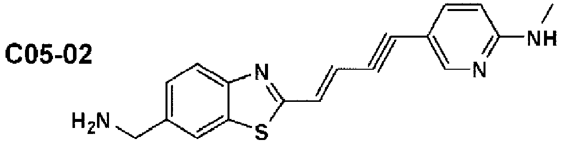

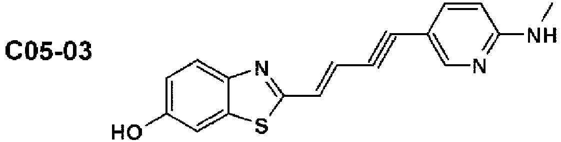

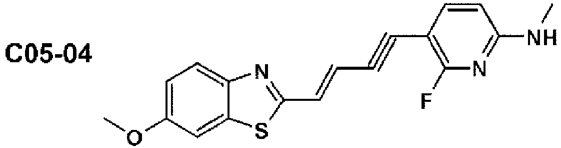

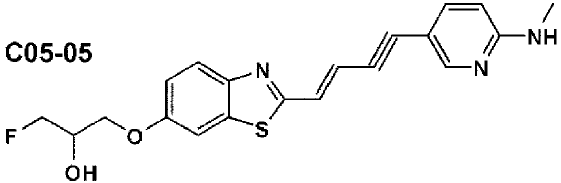

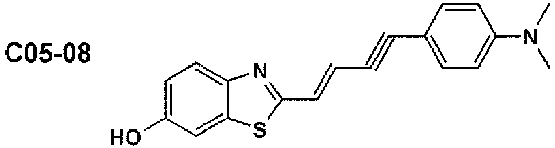

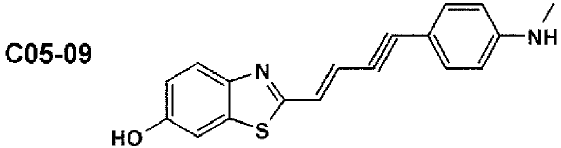

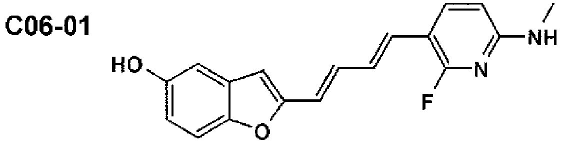

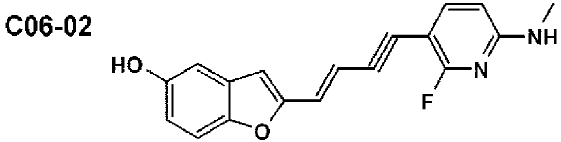

### (Preparation of BF-227)

BF-227 (2-(2-[2-dimethylaminothiazol-5-yl]ethenyl)-6-(2-[fluoro]ethoxy)benzoxazole) was that of the catalog No. NP039-0 available from NARD INSTITUTE, LTD.

### (Synthesis of PBB3)

PBB3 ((2-((1E,3E)-4-(6-(methylamino)pyridin-3-yl)buta-1,3-dienyl)benzo[d]thiazol-6-ol) was synthesized by the method disclosed in Synthetic Example 3 of Patent Literature 1.

### (Synthesis of C05-01)

C05-01 (((E)-2-(4-(2-fluoro-6-(methylamino)pyridin-3-yl)but-1-en-3-ynyl)benzo[d]thiazol-6-ol) was synthesized by the method disclosed in Synthetic Example 23 of Patent Literature 1.

### (Synthesis of C05-02)

C05-02 (((E)-5-(4-(6-(aminomethyl)benzo[d]thiazol-2-yl)but-3-en-1-ynyl)-N-methylpyridine-2-amine) was synthesized by the method disclosed in Synthetic Example 11 of Patent Literature 1.

### (Synthesis of C05-03)

C05-03 (((E)-2-(4-(6-(methylamino)pyridin-3-yl)but-1-en-3-ynyl)benzo[d]thiazol-6-ol) was synthesized by the method disclosed in Synthetic Example 10 of Patent Literature 1.

### (Synthesis of C05-05)

C05-05 (((E)-1-fluoro-3-(2-(4-(6-(methylamino)pyridin-3-yl)but-1-en-3-ynyl)benzo[d]thiazol-6-yloxy)propan-2-ol) was synthesized by the method disclosed in Synthetic Example 21 of Patent Literature 1.

### (Synthesis of C05-08)

C05-08 (((E)-2-(4-(4-(dimethylamino)phenyl)but-1-en-3-ynyl)benzo[d]thiazol-6-ol) was synthesized by the method disclosed in Synthetic Example 6 of Patent Literature 1.

### (Synthesis of C05-09)

C05-09 (((E)-2-(4-(4-(methylamino)phenyl)but-1-en-3-ynyl)benzo[d]thiazol-6-ol) was synthesized by the method disclosed in Synthetic Example 7 of Patent Literature 1.

### (Synthesis of C06-01)

C06-01 (2-((1E,3E)-4-(2-fluoro-6-(methylamino)pyridin-3-yl)buta-1,3-dienyl)benzofuran-5-ol) was synthesized by the method disclosed in Synthetic Example 24 of Patent Literature 1.

### (Synthesis of C05-04)

C05-04 (TM2) was synthesized in accordance with the following scheme.

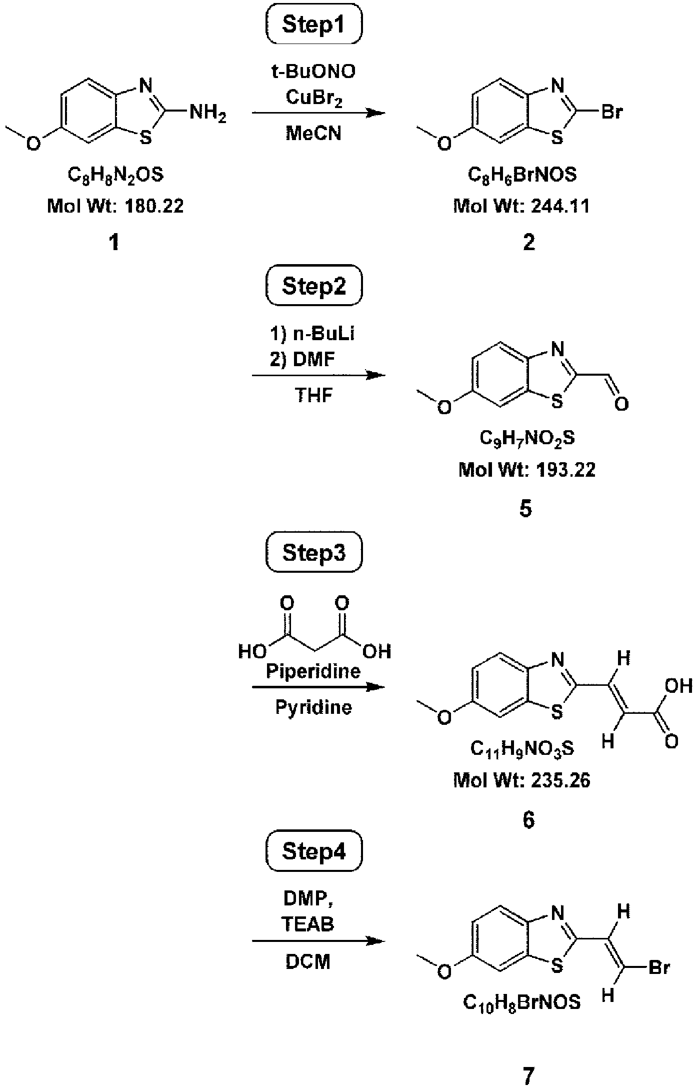

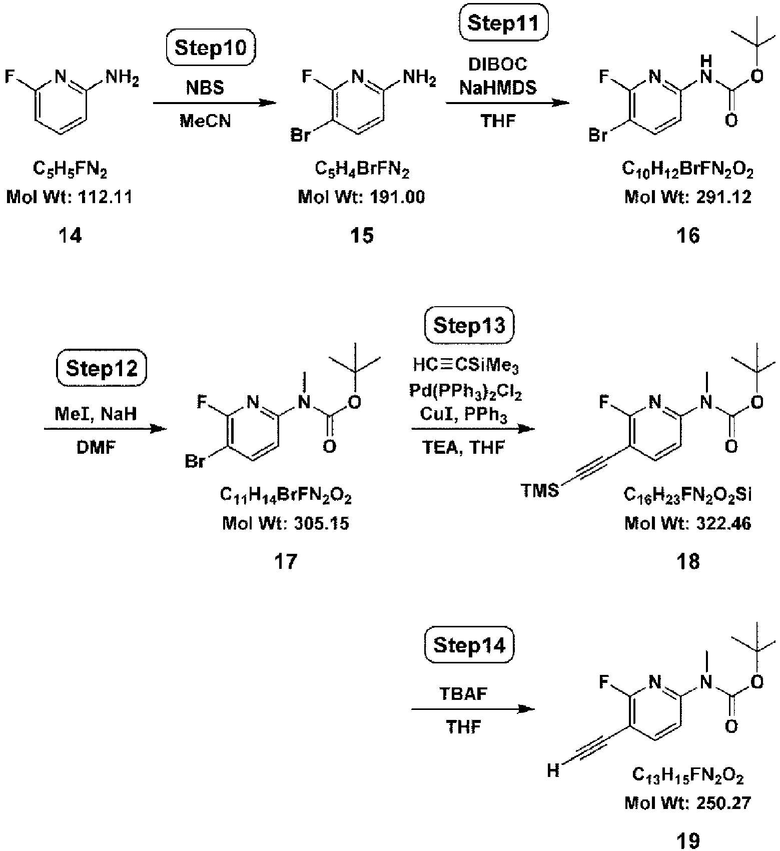

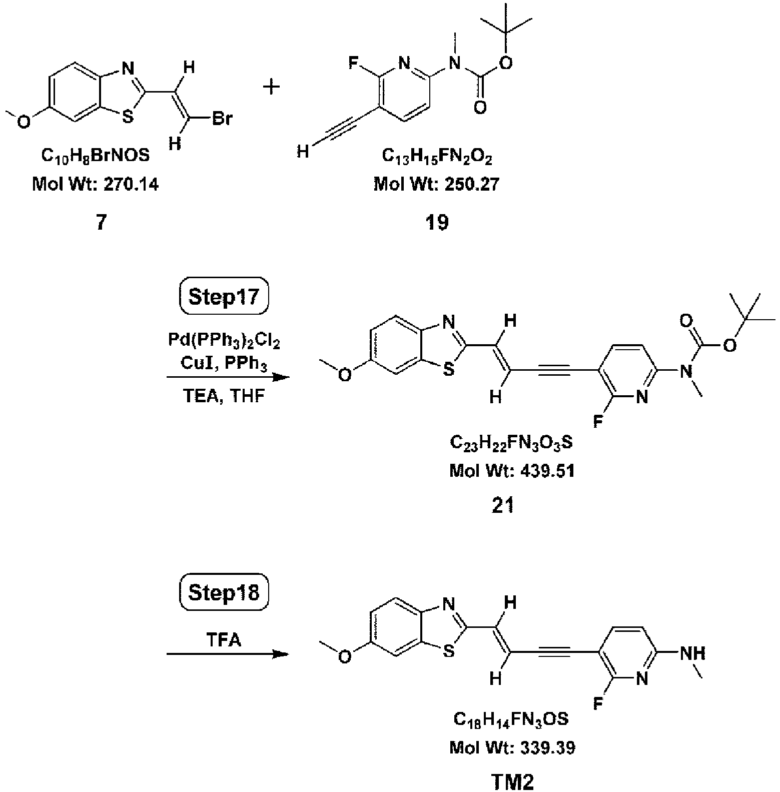

### (Step 1)

Under acetonitrile (MeCN), a compound 1 (5.01 g, available from Tokyo Chemical Industry Co., Ltd.) was caused to react with tert-butyl nitrite (t-BuONO) and CuBr₂ to obtain a compound 2 in the form of yellow oil (yield amount: 5.94 g, yield percentage: 87.5%). Note that the obtained compound 2 solidified during storage.

### (Step 2)

Under tetrahydrofuran (THF), the compound 2 (5.93 g) was caused to react with n-butyllithium (n-BuLi) and N,N-dimethylformamide (DMF) in this order to obtain a compound 5 in the form of pale yellow solid (yield amount: 3.78 g, yield percentage: 80.5%).

### (Step 3)

Under pyridine, the compound 5 (3.78 g) was caused to react with malonic acid and piperidine to obtain a compound 6 in the form of ocher solid (yield amount: 3.71 g, yield percentage: 80.6%).

### (Step 4)

Under dichloromethane (DCM), the compound 6 (4.00 g) was caused to react with Dess-Martin periodinane (DMP) and tetraethylammonium bromide (TEAB) to obtain a compound 7 in the form of yellow solid (yield amount: 1.85 g, yield percentage: 40.3%).

### (Step 10)

Under acetonitrile (MeCN), a compound 14 (5.00 g, available from Combi-Blocks) was caused to react with N-bromosuccinimide (MBS) to obtain a compound 15 in the form of pale pink solid (yield amount: 6.80 g, 79.8%).

### (Step 11)

Under tetrahydrofuran (THF), the compound 15 (6.80 g) was caused to react with di-tert-butyl dicarbonate (DIBOC) and sodium hexamethyldisilazane (NaHMDS) to obtain a compound 16 in the form of yellow oil (yield amount: 2.42 g, yield percentage: 23.3%).

### (Step 12)

Under N,N-dimethylformamide (DMF), the compound 16 (2.75 g) was caused to react with methyl iodide (MeI) and sodium hydride (NaH) to obtain a compound 17 in the form of colorless oil (yield amount: 2.82 g, yield percentage: 97.8%).

### (Step 13)

The compound 17 (2.81 g) was caused to react with trimethylsilylacetylene in a mixed solution of triethylamine (TEA) and tetrahydrofuran (THF) in the presence of bis(triphenylphosphine)palladium(II) dichloride (Pd(PPh₃)₂Cl₂), copper iodide (CuI), and triphenylphosphine (PPh₃) to obtain a compound 18 in the form of colorless oil (yield amount: 2.50 g, yield percentage: 84.2%). Note that the obtained compound 18 solidified during storage.

### (Step 14)

Under tetrahydrofuran (THF), a compound 18 (2.49 g) was caused to react with tetra-n-butylammonium fluoride (TBAF) to obtain a compound 19 in the form of pale brown oil (yield amount: 1.85 g, yield percentage: 95.7%).

### (Step 17)

The compound 7 (213.0 mg) and the compound 19 (261.8 mg) were caused to reacted with each other in a mixed solution of triethylamine (TEA) and tetrahydrofuran (THF) in the presence of bis(triphenylphosphine)palladium(II) dichloride (Pd(PPh₃)₂Cl₂), copper iodide (CuI), triphenylphosphine (PPh₃) to obtain a compound 21 in the form of pale yellow solid (yield amount: 256.3 mg, yield percentage: 74.0%).

### (Step 18)

The compound 21 (245.3 mg) was caused to react with trifluoroacetic acid (TFA) to obtain C05-04 (TM2) in the form of yellow solid (yield amount: 174.2 mg, yield percentage: 92.0%). Note that the total yield percentage was 15%. ¹H NMR and ESI MS of the obtained C05-04 are shown below.

¹H NMR (DMSO-d6, 400 MHz) δ: 2.79 (3H, brs), 3.84 (3H, s), 6.40 (1H, m), 6.93 (1H, d, J = 16.0 Hz), 7.12 (1H, m), 7.21 (1H, d, J = 16.0 Hz), 7.58-7.68 (3H, m), 7.87 (1H, m)

ESI MS: m/z found 340.09 (Calcd for C₁₈H₁₄FN₃OS [M+H]⁺ 340.09)

### (Synthesis of C06-03)

C06-03 (TM1) was synthesized in accordance with the following scheme.

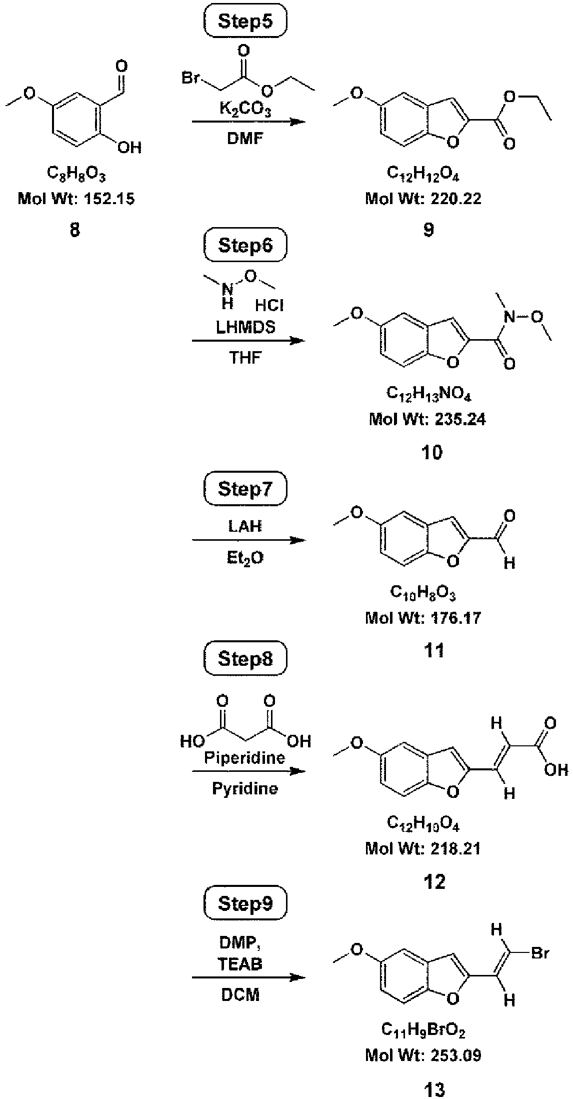

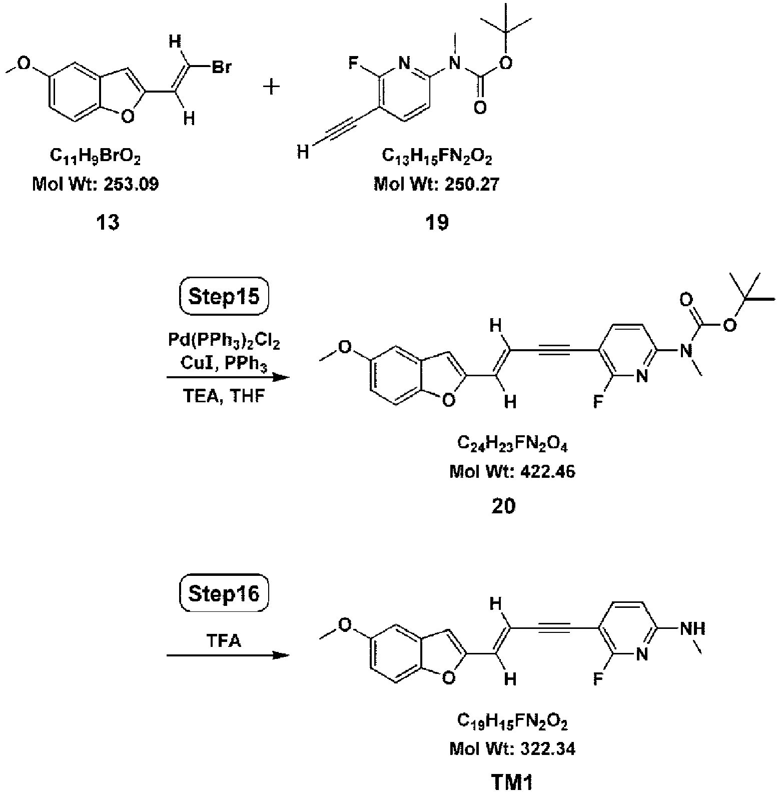

### (Step 5)

A compound 8 (24.95 g, available from Tokyo Chemical Industry Co., Ltd.) was caused to react with ethyl bromoacetate and K₂CO₃ under N,N-dimethylformamide (DMF) to obtain a compound 9 in the form of white solid (yield amount: 26.07 g, yield percentage: 72.2%).

### (Step 6)

Under tetrahydrofuran (THF), the compound 9 (5.72 g) was caused to react with N-methoxy-N-methylamine hydrochloride and lithium bis(trimethylsilyl)amide (LHMDS) to obtain a compound 10 in the form of brown oil (yield amount: 5.68 g, yield percentage: 93.0%).

### (Step 7)

The compound 10 (5.68 g) was caused to react with lithium aluminum hydride (LAH) under diethyl ether (Et₂O) to obtain a compound 11 in the form of yellow solid (yield amount: 3.80 g, yield percentage: 89.3%).

### (Step 8)

Under pyridine, the compound 11 (3.69 g) was caused to react with malonic acid and piperidine to obtain a compound 12 in the form of pale yellow solid (yield amount: 4.41 g, yield percentage: 96.5%).

### (Step 9)

Under dichloromethane (DCM), the compound 12 (4.40 g) was caused to react with Dess-Martin periodinane (DMP) and tetraethylammonium bromide (TEAB) to obtain a compound 13 in the form of pale yellow solid (yield amount: 4.49 g, yield percentage: 87.9%).

### (Step 15)

The compound 13 (596.6 mg) and the compound 19 (765.2 mg) were caused to reacted with each other in a mixed solution of triethylamine (TEA) and tetrahydrofuran (THF) in the presence of bis(triphenylphosphine)palladium(II) dichloride (Pd(PPh₃)₂Cl₂), copper iodide (CuI), triphenylphosphine to obtain a compound 20 in the form of pale yellow solid (yield amount: 375.5 mg, yield percentage: 37.7%).

### (Step 16)

The compound 20 (375.5 mg) was caused to react with trifluoroacetic acid (TFA) to obtain C06-03 (TM1) in the form of yellow solid (yield amount: 149.5 mg, yield percentage: 52.2%). Note that the total yield percentage was 10%. ¹H NMR and ESI MS of the obtained C06-03 are shown below.

¹H NMR (DMSO-d6, 400 MHz) δ: 2.78 (3H, brs), 3.78 (3H, s), 6.38 (1H, m), 6.51 (1H, d, J = 16.0 Hz), 6.91 (1H, m), 6.95 (1H, s), 6.98 (1H, d, J = 16.0 Hz), 7.14 (1H, m), 7.44-7.49 (2H, m), 7.59 (1H, m)

ESI MS: m/z found 323.12 (Calcd for C₁₉H₁₅FN₂O₂ [M+H]⁺ 323.12)

### (Synthesis of [¹⁸F]C05-01)

[¹⁸F]C05-01 was synthesized in accordance with the following scheme.

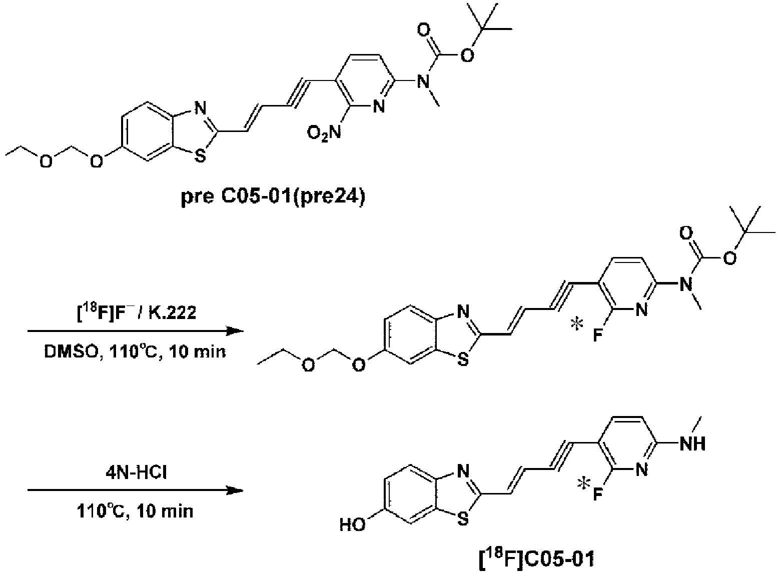

[¹⁸F]fluoride ions were eluted with a 50% acetonitrile solution (0.4 mL) containing K.222 (Kryptofix 222) (7.5 mg) and potassium carbonate (2.77 mg) and introduced into a reactor vessel, and were then heated under a nitrogen gas flow to dry and solidify a solvent. Subsequently, anhydrous acetonitrile (0.1 mL) was added for azeotropic evaporation, and the inside of the reactor vessel was sufficiently dried. The following reaction was carried out while being shielded from light. A DMSO (300 µL) solution containing tert-butyl(E)-(5-(4-(6-(ethoxymethoxy)benzo[d]thiazol-2-yl)but-3-en-1-yn-1-yl)-6-nitropyridin-2-yl)(methyl)carbamate (pre C05-01) (1 mg) synthesized by the method disclosed in Synthetic Example 38 (pre24) of Patent Literature 1 was added to the reactor vessel. The reaction mixed solution was heated at 110°C for 10 minutes. The reactor vessel was cooled down, and then 4N hydrochloric acid (0.5 mL) was added to carry out hydrolysis at 110°C for 10 minutes. The reactor vessel was cooled down, and then 1N sodium acetate (2 mL) was added and stirred, and an HPLC solvent (500 µL) was added. The mixed solution was purified by HPLC (HPLC: CAPCELL PAK C18 UG 80 10 mm × 250 mm, acetonitrile/50 mM ammonium acetate = 6/4, 4 mL/min). A fraction equivalent to [¹⁸F]C05-01 was collected in a flask containing 25% ascorbic acid (100 µL) and Tween80 (100 µL) in ethanol (300 µL), and the solvent was evaporated under reduced pressure. The residue was dissolved in physiological saline water (2.5 mL, pH7.4) to obtain [¹⁸F]C05-01 as an injection solution.

### (Synthesis of [¹¹C]C05-04)

[¹¹C]C05-04 was synthesized in accordance with the following scheme.

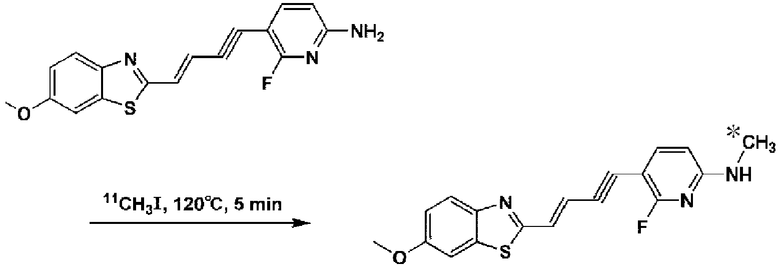

The following synthesis was carried out while being shielded from light. Iodo[¹¹C]methane was added to a dimethyl sulfoxide (DMSO) (300 µL) solution containing (E)-6-fluoro-5-(4-(6-methoxybenzo[d]thiazol-2-yl)but-3-en-1-yn-1-yl)pyridine-2-amine (pre C05-04-1) (1.5 mg) at a room temperature. The reaction mixed solution was heated at 120°C for 5 minutes. The reactor vessel was cooled down, and then an HPLC solvent (500 µL) was added. The mixed solution was purified by HPLC (HPLC: Waters Atlantis prep T3 10 mm × 150 mm, acetonitrile/50 mM ammonium acetate = 4/6, 5 mL/min). A fraction equivalent to [¹¹C]C05-04 was collected in a flask containing 25% ascorbic acid (100 µL) and Tween80 (75 µL) in ethanol (300 µL), and the solvent was evaporated under reduced pressure. The residue was dissolved in physiological saline water (3 mL, pH7.4) to obtain [¹¹C]C05-04 as an injection solution. Note that pre C05-04-1 was synthesized by a method similar to the synthesis of the foregoing C05-04 (TM2).

### (Synthesis of [¹⁸F]C05-04)

[¹⁸F]C05-04 was synthesized in accordance with the following scheme.

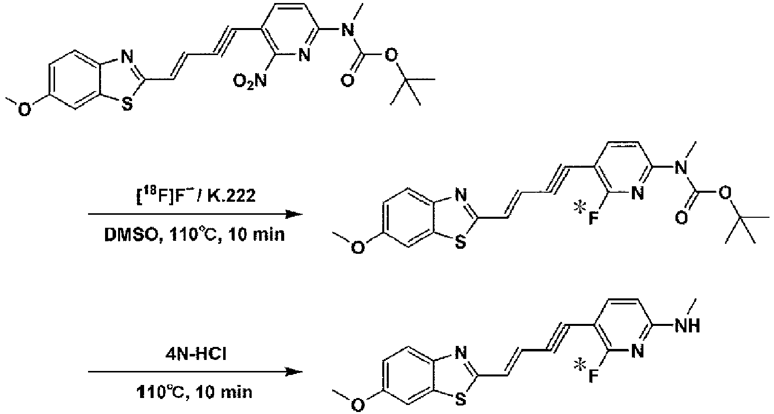

[¹⁸F]fluoride ions were eluted with a 50% acetonitrile solution (0.4 mL) containing K.222 (Kryptofix 222) (7.5 mg) and potassium carbonate (2.77 mg) and introduced into a reactor vessel, and were then heated under a nitrogen gas flow to dry and solidify a solvent. Subsequently, anhydrous acetonitrile (0.1 mL) was added for azeotropic evaporation, and the inside of the reactor vessel was sufficiently dried. The following reaction was carried out while being shielded from light. A DMSO (300 µL) solution containing tert-butyl(E)-(5-(4-(6-methoxybenzo[d]thiazol-2-yl)but-3-en-1-yn-1-yl)-6-nitropyridin-2-yl)(methyl)carbamate (pre C05-04-2) (1.5 mg) was added to the reactor vessel. The reaction mixed solution was heated at 110°C for 10 minutes. The reactor vessel was cooled down, and then 4N hydrochloric acid (0.3 mL) was added to carry out hydrolysis at 110°C for 10 minutes. The reactor vessel was cooled down, and then 2N sodium acetate (0.6 mL) was added and stirred, and an HPLC solvent (500 µL) was added. The mixed solution was purified by HPLC (HPLC: Waters Atlantis prep T3 10 mm × 150 mm, acetonitrile/50 mM ammonium acetate = 4/6, 5 mL/min). A fraction equivalent to [¹⁸F]C05-04 was collected in a flask containing 25% ascorbic acid (100 µL) and Tween80 (75 µL) in ethanol (300 µL), and the solvent was evaporated under reduced pressure. The residue was dissolved in physiological saline water (3 mL, pH7.4) to obtain [¹⁸F]C05-04 as an injection solution. Note that pre C05-04-2 was synthesized by a method similar to the synthesis of the foregoing C05-04 (TM2).

### (Synthesis of [¹¹C]C06-03)

[¹¹C]C06-03 was synthesized in accordance with the following scheme.

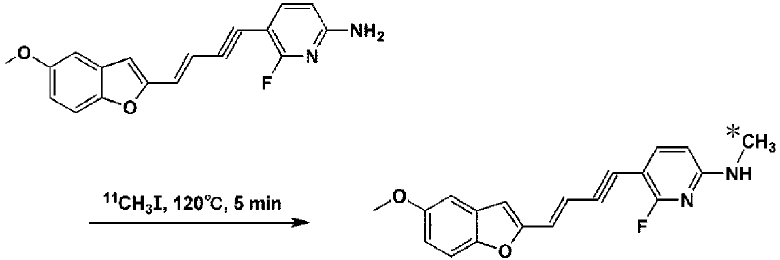

The following synthesis was carried out while being shielded from light. Iodo[¹¹C]methane was added to a dimethyl sulfoxide (DMSO) (300 µL) solution containing (E)-6-fluoro-5-(4-(5-methoxybenzofuran-2-yl)but-3-en-1-yn-1-yl)pyridine-2-amine (pre C06-03-1) (1.5 mg) at a room temperature. The reaction mixed solution was heated at 120°C for 5 minutes. The reactor vessel was cooled down, and then an HPLC solvent (500 µL) was added. The mixed solution was purified by HPLC (HPLC: Waters Atlantis prep T3 10 mm × 150 mm, acetonitrile/50 mM ammonium acetate = 4/6, 5 mL/min). A fraction equivalent to [¹¹C]C06-03 was collected in a flask containing 25% ascorbic acid (100 µL) and Tween80 (75 µL) in ethanol (300 µL), and the solvent was evaporated under reduced pressure. The residue was dissolved in physiological saline water (3 mL, pH7.4) to obtain [¹¹C]C06-03 as an injection solution. Note that pre C06-03-1 was synthesized by a method similar to the synthesis of the foregoing C06-03 (TM1).

### (Synthesis of [¹⁸F]C06-03)

[¹⁸F]C06-03 was synthesized in accordance with the following scheme.

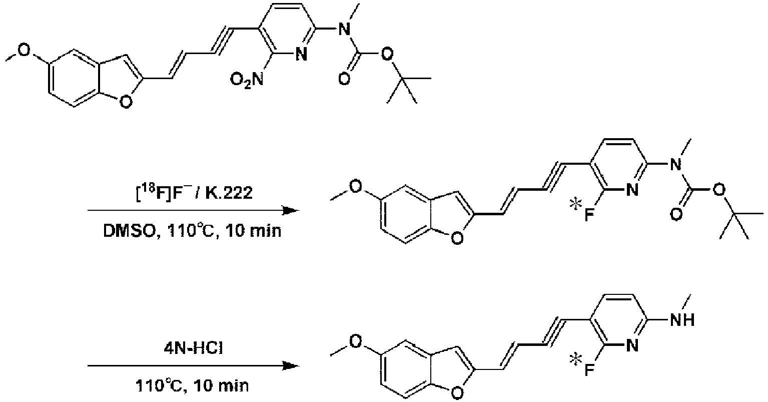

[¹⁸F]fluoride ions were eluted with a 50% acetonitrile solution (0.4 mL) containing K.222 (Kryptofix 222) (7.5 mg) and potassium carbonate (2.77 mg) and introduced into a reactor vessel, and were then heated under a nitrogen gas flow to dry and solidify a solvent. Subsequently, anhydrous acetonitrile (0.1 mL) was added for azeotropic evaporation, and the inside of the reactor vessel was sufficiently dried. The following reaction was carried out while being shielded from light. A DMSO (300 µL) solution containing tert-butyl(E)-(5-(4-(5-methoxybenzofuran-2-yl)but-3-en-1-yn-1-yl)-6-nitropyridin-2-yl)(methyl)carbamate (pre C06-03-2) (1.5 mg) was added to the reactor vessel. The reaction mixed solution was heated at 110°C for 10 minutes. The reactor vessel was cooled down, and then 4N hydrochloric acid (0.3 mL) was added to carry out hydrolysis at 110°C for 10 minutes. The reactor vessel was cooled down, and then 2N sodium acetate (0.6 mL) was added and stirred, and an HPLC solvent (500 µL) was added. The mixed solution was purified by HPLC (HPLC: Waters Atlantis prep T3 10 mm × 150 mm, acetonitrile/50 mM ammonium acetate = 4/6, 5 mL/min). A fraction equivalent to [¹⁸F]C06-03 was collected in a flask containing 25% ascorbic acid (100 µL) and Tween80 (75 µL) in ethanol (300 µL), and the solvent was evaporated under reduced pressure. The residue was dissolved in physiological saline water (3 mL, pH7.4) to obtain [¹⁸F]C06-03 as an injection solution. Note that C06-03-2 was synthesized by a method similar to the synthesis of the foregoing C06-03 (TM1).

### <Optical imaging of human brain>

### (Dissected brain tissue)

Postmortem human brains were obtained from autopsies carried out with respect to a dementia with Lewy bodies (DLB) and Alzheimer's disease (AD) patients.

A frozen DLB tissue was sliced with a thickness of 20 µm in a cryostat (HM560, Carl Zeiss).

Moreover, an AD brain tissue was fixed in 10% neutral buffered formalin, embedded in a paraffin block, and sliced with a thickness of 6 µm.

### (In vitro fluorescence microscope measurement)

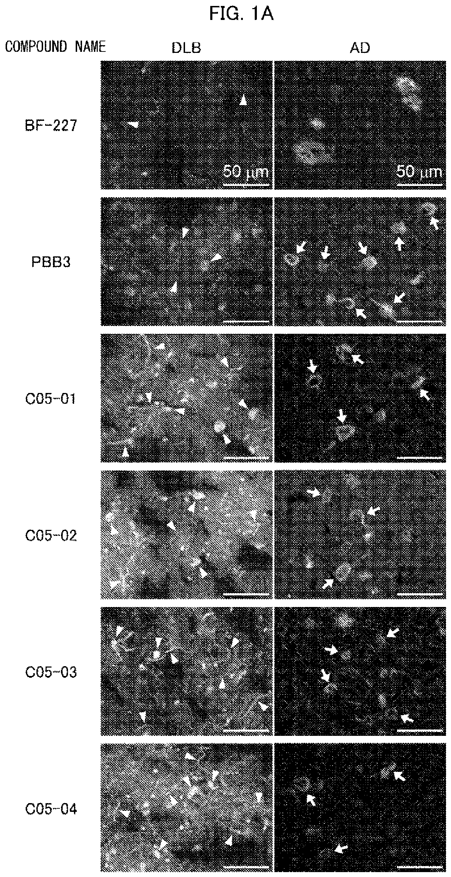

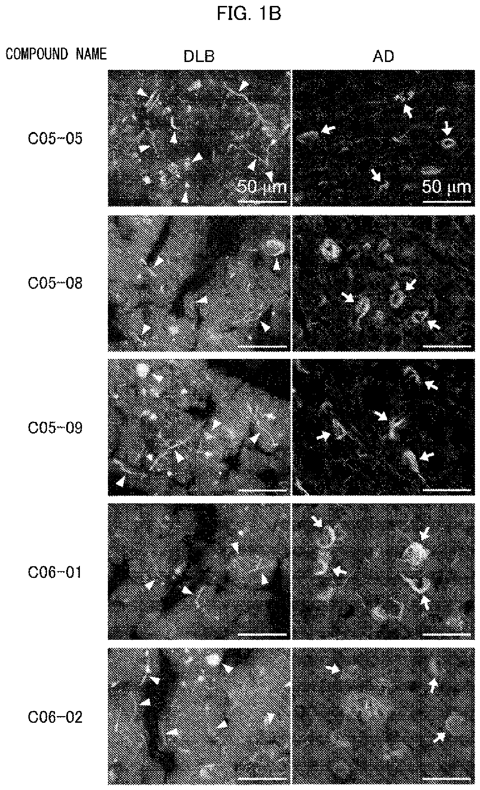

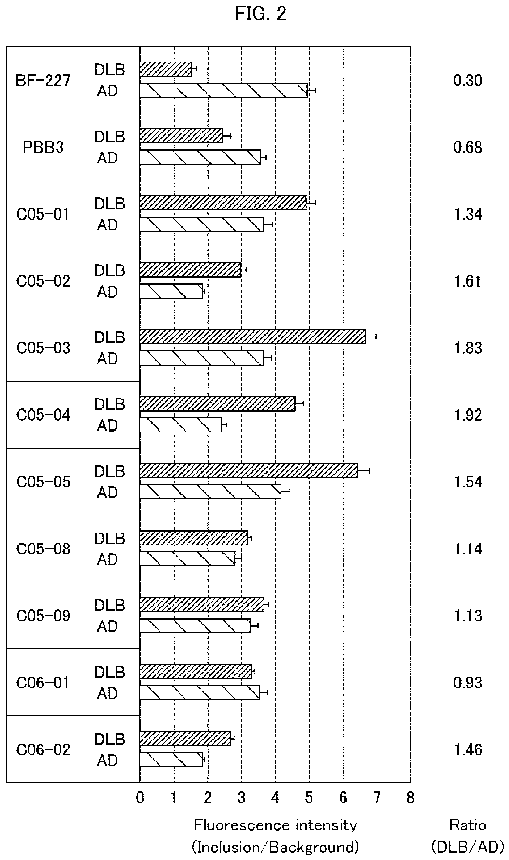

Postfixed fresh frozen sections of the amygdala tissue of the DLB patient and formalin fixed, paraffin embedded sections of a deparaffinized middle frontal gyrus tissue of the AD patient were used. Thirty µM of each of the above compounds and brain sections were incubated in a 50% ethanol solution for 30 minutes at room temperature. The sections were then washed twice, i.e., washed with a 50% ethanol solution for 5 minutes and with ultrapure water for 3 minutes. After mounting the sections using a mounting medium (VECTASHIELD H-1000, Vector Laboratories), images of lesion-enriched areas on the sections were obtained using a fluorescence microscope (DM4000, Leica). Fluorescent images are shown in Fig. 1 (Figs. 1A and 1B). Analysis software (Image J) was used to quantify the fluorescence of lesion-enriched and lesion-free (background) areas. Results are shown in Fig. 2.

As shown in Figs. 1 and 2, it was confirmed that the compounds represented by the formula (I), i.e., C05-01, C05-02, C05-03, C05-04, C05-05, C05-08, C05-09 and C06-02 bound to the α-synuclein aggregate formed in the brain of the DLB patient with reactivity equal to or greater than that of PBB3. It was also confirmed that the binding of the compounds represented by the formula (I) to the α-synuclein aggregate was stronger than the binding to the aggregate of tau or amyloid-β formed in the brain of the AD patient. In addition, in vitro fluorescence microscope measurement was carried out on the brain of an MSA patient in a manner similar to that for the brain of the DLB patient, and similar results were obtained.

Moreover, it was confirmed that the compounds represented by the formula (I) had higher selectivity for the α-synuclein aggregate than to the aggregate formed in the AD patient brain, as compared with C06-01 which is a compound having a butadiene bond. Note that it was confirmed that BF-227 reported as a PET probe for an α-synuclein lesion mainly bound to the amyloid-β aggregate of AD, and that the binding to the α-synuclein aggregate was weaker than the compounds represented by the formula (I).

### <Optical imaging of mouse brain>

### (Preparation of α-synuclein fibril-injected mouse model)

First, murine α-synuclein was expressed in and extracted from E. coli as a recombinant protein, and incubated in vitro to form an insoluble α-synuclein aggregate.

Then, after the α-synuclein aggregate was inoculated into the striatum of a mouse, the α-synuclein aggregate was propagated to surrounding areas via the neural circuitry, and an α-synuclein lesion was observed in the neocortex several months later.

Note that the α-synuclein aggregate was inoculated into the striatum of the mouse with the following manner. First, hair on the head of 9-week-old C57BL/6 male mouse anesthetized with 1.5% (v/v) isoflurane was removed, and the scalp was disinfected with isodine, followed by application of xylocaine and incision in the scalp to expose the skull. Then, a hole was drilled in the skull at a position of 0.05 mm posterior to the bregma and 2 mm lateral to the midline, and 3 µl of an α-synuclein fiber solution (murine α-synuclein fiber (4 mg/ml) in saline) was injected into a position 2 µm ventral to the brain surface using a glass pipette, and then the scalp was returned and sutured.

### (In vitro fluorescence microscope measurement)

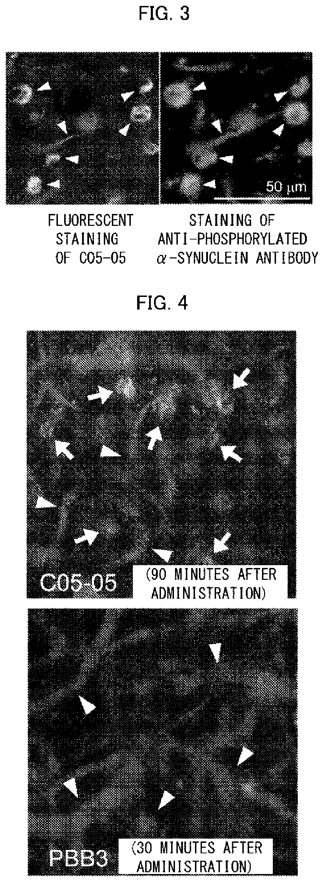

The brain of the α-synuclein fibril-injected mouse was extracted, sectioned, and analyzed by fluorescent staining. Specifically, the α-synuclein fibril-injected mouse brain sections and 30 µM of the compound were incubated in a 20% ethanol solution for 30 minutes at room temperature. The sections were then washed twice, i.e., washed with a 20% ethanol solution for 5 minutes and with ultrapure water for 3 minutes. After mounting the sections using a mounting medium (VECTASHIELD H-1000), images of α-synuclein aggregate-enriched areas on the sections were obtained using a fluorescence microscope (DM4000). The same sections were washed using a phosphate buffer solution and then treated with an autoclave for the antigen retrieval. Immunohistochemical staining with an anti-phosphorylated α-synuclein monoclonal antibody (pS129, abcam, ab59264) (1:1000) was carried out, and after mounting the sections using a mounting medium (VECTASHIELD H-1000), images of the same areas were obtained using the fluorescence microscope (DM4000). The results are shown in Fig. 3. In Fig. 3, the right side shows the staining with the anti-phosphorylated α-synuclein antibody and the left side shows the fluorescent staining with C05-05.

The results in Fig. 3 show that C05-05, which is the compound represented by the formula (I), binds to the lesion composed of the phosphorylated α-synuclein.

### (In vivo two-photon laser scanning fluorescence microscopy)

A model mouse was anesthetized with 1.5% (v/v) isoflurane 6 weeks after the injection of the α-synuclein fiber solution, and a cranial window was provided according to the Seylaz-Tomita method (Tomita et al. J Cereb Blood Flow Metab 25, 858-67, 2005). The mouse was anesthetized with 1.5% (v/v) isoflurane 2 weeks after the generation of the cranial window, and 50 µl of a solution of DMSO/physiological saline water = 1:1 containing 0.05% of C05-05 or PBB3 was administered via the tail vein. The mouse was fixed under a two-photon laser fluorescence microscope 90 minutes after administration of C05-05 or 30 minutes after administration of PBB3, and 100 µl of a saline solution containing 5 mM of Sulforhodamine 101 was administered intraperitoneally, followed by intravital two-photon fluorescence imaging at an excitation wavelength of 900 nm. The detection wavelengths for C05-05 and PBB3 were set at 500 nm to 550 nm, and detection wavelengths for Sulforhodamine 101 were set at 573 nm to 648 nm. The results are shown in Fig. 4.

From the results shown in Fig. 4, it was observed by the intravital two-photon laser fluorescence microscope that, when C05-05, which is the compound represented by the formula (I), was intravenously injected, the compound entered the brain of the living body, and thus into neurons, and bound to individual α-synuclein lesions. Therefore, even in a case where the density or total number of lesions is small and it is difficult to detect the lesions by PET, the lesions can be detected by biofluorescence imaging. In contrast, no lesion was detected by intravenous injection of PBB3, and it was confirmed that C05-05, which is the compound represented by the formula (I), achieved higher contrast than PBB3 in detecting lesions.

### <In vivo positron emission tomography (PET) imaging>

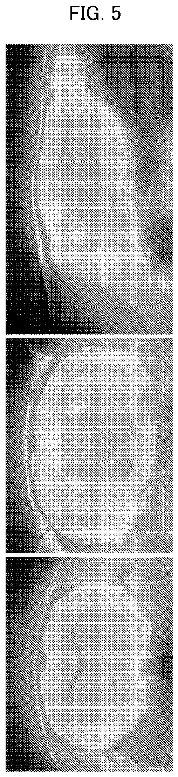

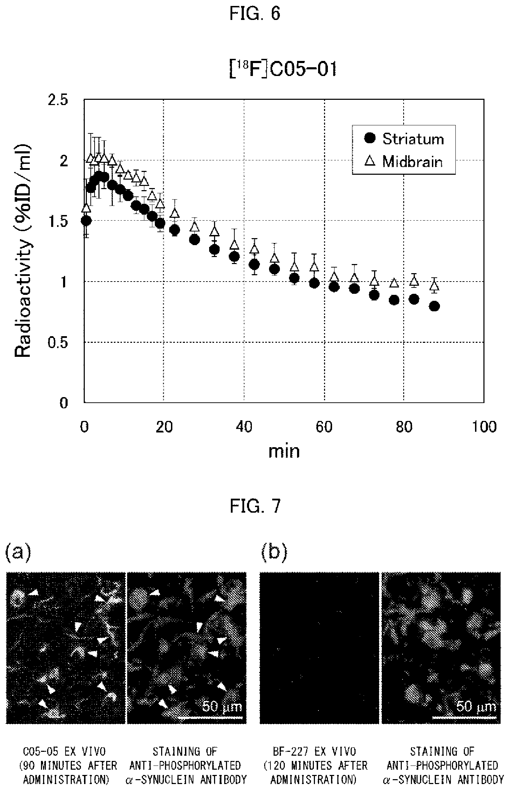

PET scanning was carried out with use of a microPET Focus 220 animal scanner (Siemens Medical Solutions), which provided 95 slices having a thickness of 0.851 mm (between centers), a 19.0-cm axial field of view (FOV) and a 7.6-cm cross-sectional FOV. Before scanning, C57BL/6 mice were anesthetized with 1.5% (v/v) of isoflurane. An emission scan was carried out immediately after intravenous injection of [¹⁸F]C05-01 (compound in which C05-01 was labeled with a positron-emitting radionuclide) (24.3±0.7 MBq), for 90 minutes, in 3D list mode, with an energy window of 350-750 keV. The injection of the radioactive compound and the scan were carried out under dim light so as to avoid photoracemization of the compound. The entire list mode data were sorted into 3D sinograms, and then converted into 2D sinograms by Fourier-rebining (frame: 10×1, 6×5, and 5×10 minutes). After the injection of the radioactive compound, summation images at 30 to 60 minutes and at 60 to 90 minutes were obtained by maximum a posteriori reconstruction. Moreover, dynamic images were reconstructed by filtered back projection, using a 0.5-mm Hanning filter. A volume of interest (VOI) was defined in the striatum and midbrain, using PMOD image analysis software (PMOD Technologies), with reference to an MRI template. The results are shown in Figs. 5 and 6.

As a result of labeling C05-01, which is the compound represented by the formula (I), with the positron-emitting radionuclide, intravenously injecting C05-01 into a normal mouse, and PET scan of the mouse, it was confirmed that C05-01 had adequate brain-entering property and clearance rate from the brain, and that C05-01 exhibited desirable characteristics as a probe for imaging the α-synuclein aggregate in the living brain by PET (see Figs. 5 and 6). Fig. 5 shows images of two coronal sections and one midline sagittal section of the normal mouse brain during a period of 1 to 30 minutes after the intravenous injection of [¹⁸F]C05-01, and are superimposed on standard brain MRI images. Fig. 6 shows time-radioactivity curves in the striatum and midbrain after intravenous injection of [¹⁸F]C05-01.

### <Optical imaging of mouse brain>

### (Ex vivo imaging)

A model mouse at 6 weeks after the injection of the α-synuclein fiber solution was anesthetized with 1.5% (v/v) isoflurane in a manner similar to that described in "(Preparation of α-synuclein fibril-injected mouse model)" above, and C05-05 (1.66 mg/kg) or BF-227 (1.66 mg/kg) was intraperitoneally administered.

The brains were collected and frozen 90 minutes after the administration of C05-05 (or 120 minutes after the administration of BF-227), and frozen sections with the thickness of 20 µm were prepared in a cryostat (HM560). After mounting the sections using a mounting medium (VECTASHIELD H-1000), images of α-synuclein aggregate-enriched areas on the sections were obtained using a fluorescence microscope (DM4000).

Moreover, the same sections were immersed and fixed overnight in a phosphate buffer solution containing 4% paraformaldehyde. The brain after fixation was washed under running water and then treated with an autoclave for the antigen retrieval. Immunohistochemical staining with an anti-phosphorylated α-synuclein monoclonal antibody (pS129, abcam, ab59264) (1:1000) was carried out, and after mounting the sections using a mounting medium (VECTASHIELD H-1000), images of the same areas were obtained using the fluorescence microscope (DM4000). The results are shown in Fig. 7. Fig. 7(a) contains images showing the case where C05-05 was administered, and Fig. 7(b) is images showing the case where BF-227 was administered.

From the results shown in Fig. 7, it was observed by the ex vivo imaging that, when C05-05 was intravenously injected into the α-synuclein fibril-injected mouse, the compound entered the brain, and thus neurons, and bound to individual α-synuclein lesions. In contrast, no lesion was detected by intravenous injection of BF-227, and it was confirmed that C05-05, which is the compound represented by the formula (I), achieves higher contrast than BF-227 in detecting lesions.

### <In vivo positron emission tomography (PET) imaging>

PET scanning was carried out with use of a micro-PET Focus 220 animal scanner (Siemens Medical Solutions), which provided 95 slices having a thickness of 0.851 mm (between centers), a 19.0-cm axial field of view (FOV) and a 7.6-cm cross-sectional FOV. Before scanning, α-synuclein fibril-injected mice and physiological saline water-injected mice (control) were anesthetized with 1.5% (v/v) of isoflurane. An emission scan was carried out immediately after intravenous injection of [¹⁸F]C05-05 (compound in which C05-05 was labeled with a positron-emitting radionuclide) (30.8±0.4 MBq), for 120 minutes, in 3D list mode, with an energy window of 350-750 keV. The injection of the radioactive compound and the scan were carried out under dim light so as to avoid photoracemization of the compound. The entire list mode data was sorted into 3D sinograms, and then converted into 2D sinograms by Fourier-rebining (frame: 10×1, 6×5, and 5×10 minutes). After the injection of the radioactive compound, summation images at 0 to 30 minutes, 30 to 60 minutes, 60 to 90 minutes, and 90 to 120 minutes were obtained by maximum a posteriori reconstruction. Moreover, dynamic images were reconstructed by filtered back projection, using a 0.5-mm Hanning filter. A volume of interest (VOI) was defined in the striatum and cerebellum, using PMOD image analysis software (PMOD Technologies), with reference to an MRI template.

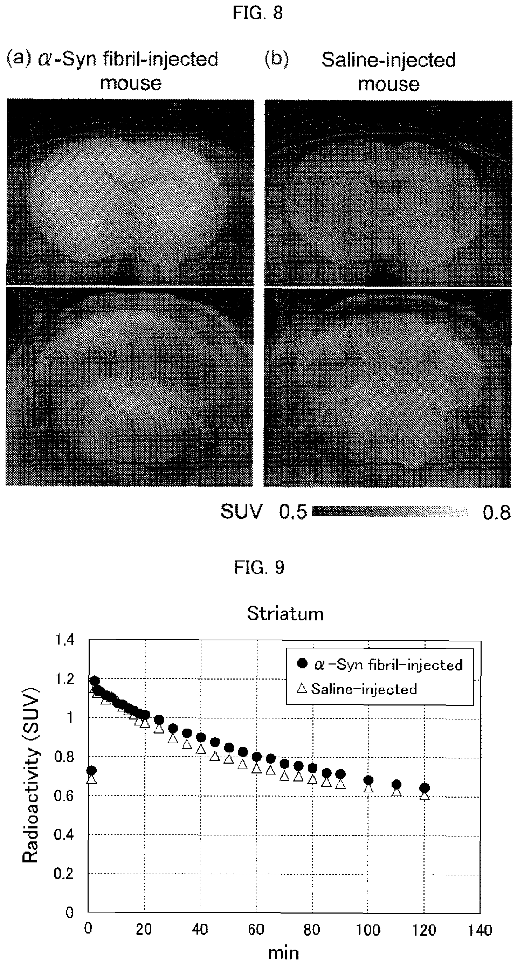

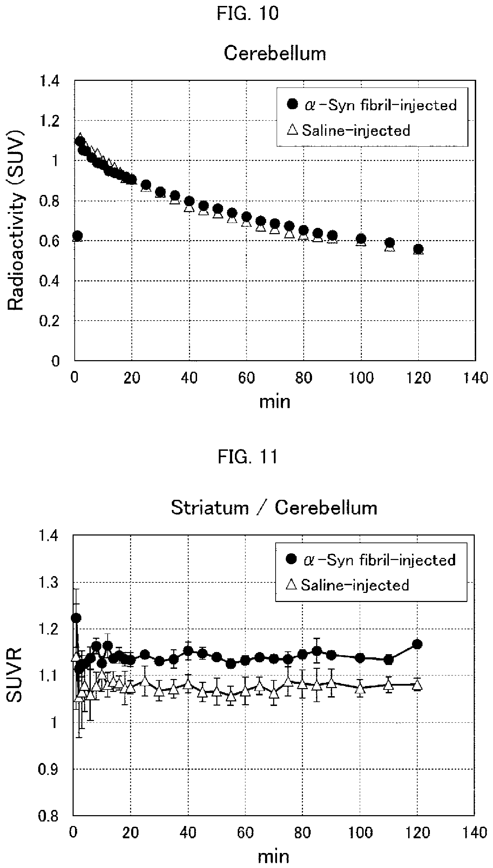

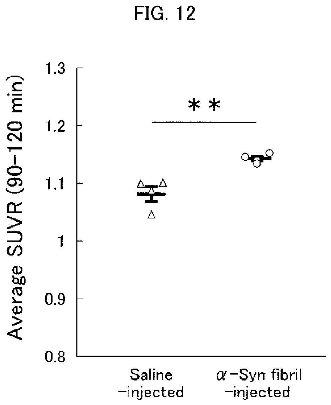

The results are shown in Figs. 8 through 12. Fig. 8(a) shows the results for the α-synuclein fiber solution-injected model mouse observed 60 to 90 minutes after the intravenous injection of [¹⁸F]C05-05. Fig. 8(b) shows the results for the saline-injected mouse. Images of coronal sections of the brains including the striata are shown in the upper row, and coronal sections of the brains including the cerebellum are shown in the lower row, and those images are superimposed on standard brain MRI images. Fig. 9 shows the time-radioactivity curves after the intravenous injection of [¹⁸F]C05-05 in the striatum, and Fig. 10 shows the time-radioactivity curves after intravenous injection of [¹⁸F]C05-05 in the cerebellum. Fig. 11 shows the temporal changes of standardized uptake value ratio (SUVR) of the striatum against the cerebellum after the intravenous injection of [¹⁸F]C05-05. Fig. 12 shows a comparison of the average SUVR during a period from 90 to 120 minutes between the α-synuclein fiber solution-injected model and saline-injected mice (n=4, **p<0.005 by Student's t-test, mean±SEMs).

C05-05, which is the compound represented by the formula (I), was labeled with the positron-emitting radionuclide and intravenously injected into the α-synuclein fiber solution-injected model mouse and into the saline-injected mouse (control). Then, a PET scan was carried out, and the compound exhibited adequate brain-entering property and clearance rate from the brain. Moreover, in the α-synuclein fiber solution-injected model mouse, clear accumulation of [¹⁸F]C05-05 on the striatum that was rich in α-synuclein lesions was seen, and it was confirmed that [¹⁸F]C05-05 enabled imaging of the α-synuclein aggregate with PET.

### <Inhibitory effect on α-synuclein aggregation by compound represented by formula (I)>

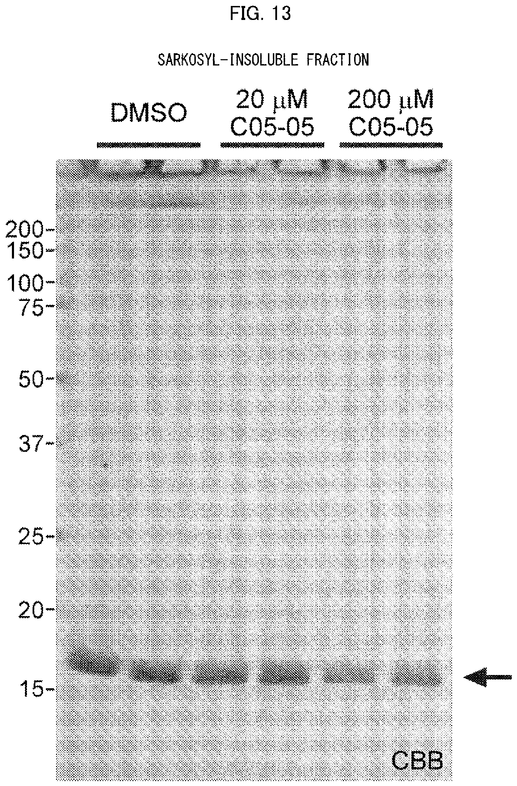

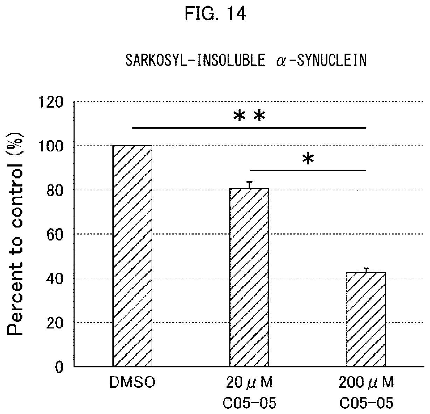

First, A53T mutant human α-synuclein was expressed in and extracted from E. coli as a recombinant protein, and incubated in vitro to prepare α-synuclein aggregate seeds. To 0.02 µg of the α-synuclein aggregate seeds, C05-05 (in DMSO solution) was added at a final concentration of 20 µM, or 200 µM and stirred. An experiment in which only DMSO was added was conducted as a control experiment. These were suspended with 50 µL of A53T mutant human α-synuclein monomer solution at a concentration of 1 mg/ml and then shaken at 37°C for 1 day. Ten µL of the suspension was mixed with 90 µL of 1% sarkosyl and ultracentrifuged at 100 Krpm for 20 minutes, and a resulting precipitate was subjected to electrophoresis, followed by CBB staining of the gel. Images of the gels were obtained using an imager, and quantification of bands was carried out using analysis software (Image J). The results are shown in Figs. 13 and 14. Fig. 13 shows results of observing insoluble α-synuclein aggregates (indicated by the arrow) contained in sarkosyl-insoluble fractions at respective conditions, and Fig. 14 shows results of quantifying these aggregates in comparison to the control experiment (DMSO) which was considered as 100%.

As shown in Fig. 14, when C05-05 at the final concentration of 20 µM was added to the α-synuclein aggregate seeds, the formation of insoluble α-synuclein aggregate was reduced by approximately 20% as compared with the control experiment. When C05-05 at the final concentration of 200 µM was added, the formation of insoluble α-synuclein aggregate was reduced by approximately 60% as compared with the control experiment. These results indicate that C05-05 has an inhibitory effect on the α-synuclein aggregation. (ANOVA F (2, 3) = 51.57, p=0.0048, ^{∗}p<0.05 and ^{∗∗}p<0.005 by Bonferroni's post hoc test, mean±SEMs).

## Claims

1. An α-synuclein aggregate binding agent, comprising a compound represented by the following formula (I), a pharmaceutically acceptable salt thereof, or a solvate thereof: 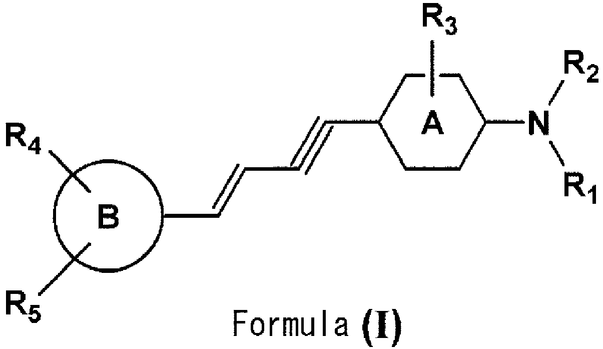 in the formula (I),
R₁ and R₂ are each independently selected from the group consisting of hydrogen, alkyl, alkenyl, acyl, and hydroxyalkyl;
R₃ is hydrogen or halogen;
the ring A is a benzene or pyridine ring;
the ring B is represented by the following formula (i) or (ii): 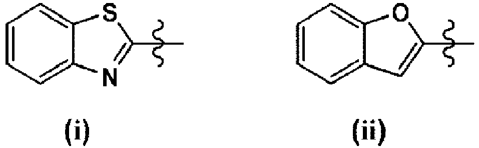
R₄ and R₅ are each independently selected from the group consisting of hydrogen, hydroxy, alkoxy, haloalkoxy, halohydroxyalkoxy, and aminoalkyl.

2. The binding agent as set forth in claim 1, wherein the ring A is a pyridine ring.

3. The binding agent as set forth in claim 1 or 2, wherein the ring B is represented by the formula (i).

4. The binding agent as set forth in claim 1, wherein the compound represented by the formula (I) is selected from the following group:

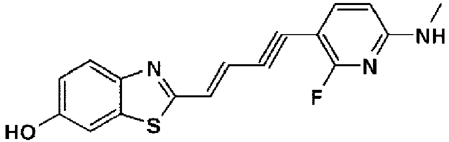

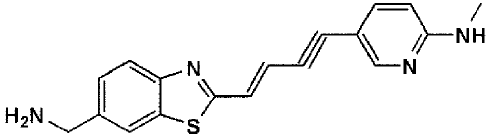

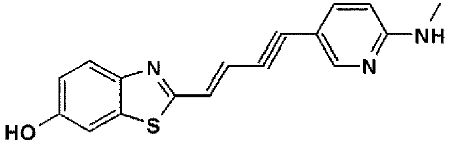

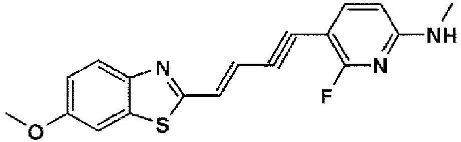

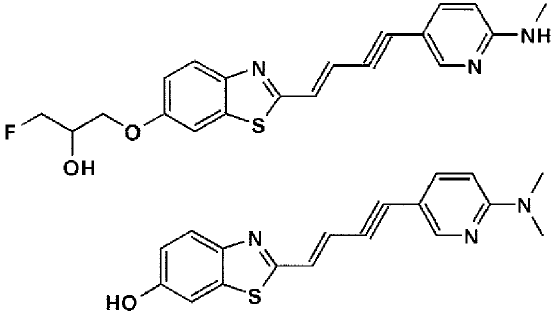

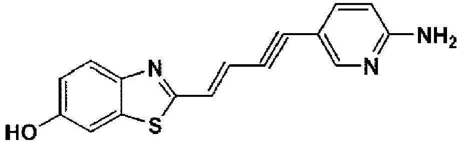

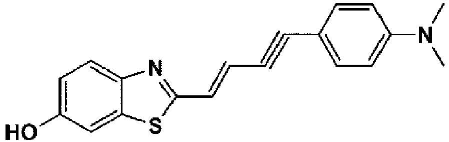

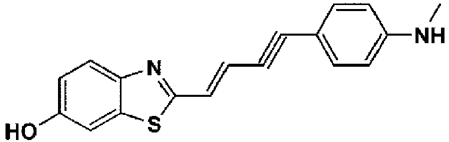

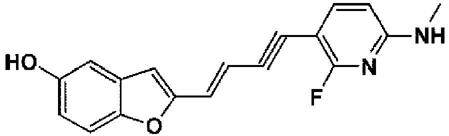

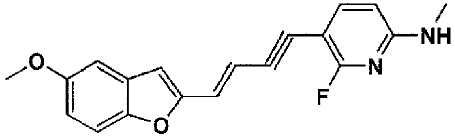

5. The binding agent as set forth in any one of claims 1 through 4, wherein, in the compound represented by the formula (I), one or more atoms are radioisotopes thereof.

6. A composition for optical imaging of an α-synuclein aggregate, said composition comprising a binding agent recited in any one of claims 1 through 4.

7. A composition for radiological imaging of an α-synuclein aggregate, said composition comprising a binding agent recited in claim 5.

8. A diagnostic agent for a disease associated with an α-synuclein aggregate or a companion diagnostic agent for treating or preventing the disease, comprising a binding agent recited in any one of claims 1 through 5.

9. A diagnostic kit for a disease associated with a substance accumulated in the brain, said diagnostic kit comprising:
a binding agent recited in any one of claims 1 through 5; and
at least one compound selected from 2-((1E,3E)-4-(6-(methylamino)pyridin-3-yl)buta-1,3-dienyl)benzo[d]thiazol-6-ol, 2-((1E,3E)-4-(6-((11)C-methylamino)pyridin-3-yl)buta-1,3-dienyl)benzo[d]thiazol-6-ol, 1-fluoro-3-(2-((1E,3E)-4-(6-(methylamino)pyridin-3-yl)buta-1,3-dienyl)benzo[d]thiazol-6-yloxy)propan-2-ol, and 1-(18)F-fluoro-3-(2-((1E,3E)-4-(6-(methylamino)pyridin-3-yl)buta-1,3-dienyl)benzo[d]thiazol-6-yloxy)propan-2-ol.

10. A method for carrying out optical imaging of an α-synuclein aggregate in the brain, said method comprising the step of:
externally irradiating the brain of a living subject, to which a binding agent recited in any one of claims 1 through 4 has been administered, with light having the first wavelength, and then detecting light which is emitted from the brain and has the second wavelength different from the first wavelength.

11. A method for carrying out radiological imaging of an α-synuclein aggregate in the brain, said method comprising the step of:
detecting radioactivity that is emitted from the brain of a living subject to which a binding agent recited in claim 5 has been administered.

12. A method of screening for a therapeutic or preventive agent for a disease associated with an α-synuclein aggregate in the brain, said method comprising the step of:
selecting a candidate substance based on a difference, which has been caused by administration of the candidate substance to the subject, in quantity and/or distribution of light or radioactivity which are detected in a method recited in claim 10 or claim 11.

13. A method for carrying out quantification or determination of accumulation of an α-synuclein aggregate in the brain, said method comprising the step of:
detecting radioactivity that is emitted from the brain of a living subject to which a binding agent recited in claim 5 has been administered,
the quantification or determination being carried out based on a quantity and/or distribution of radioactivity which has been detected.

14. A method for classification and detection of a substance accumulated in the brain, said method comprising:
the first step of detecting radioactivity that is emitted from the brain of a living subject to which a binding agent recited in claim 5 has been administered; and
the second step of detecting radioactivity that is emitted from the brain of the subject to which at least one compound selected from 2-((1E,3E)-4-(6-((11)C-methylamino)pyridin-3-yl)buta-1,3-dienyl)benzo[d]thiazol-6-ol and 1-(18)F-fluoro-3-(2-((1E,3E)-4-(6-(methylamino)pyridin-3-yl)buta-1,3-dienyl)benzo[d]thiazol-6-yloxy)propan-2-ol has been administered at a time point which is different from that of the first step,
the determination being carried out based on data of a quantity and/or distribution of the radioactivity detected in the first step and on data of a quantity and/or distribution of the radioactivity detected in the second step.

15. An α-synuclein aggregation inhibitor, comprising a compound represented by the following formula (I), a pharmaceutically acceptable salt thereof, or a solvate thereof: 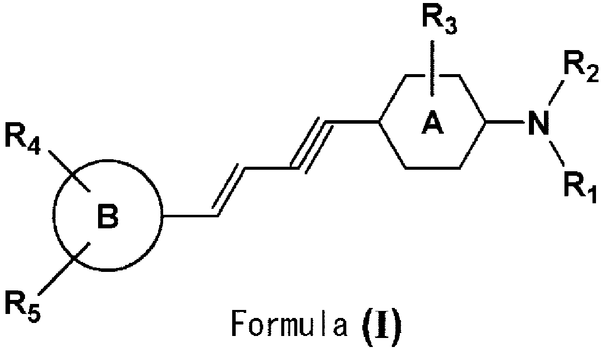 in the formula (I),
R₁ and R₂ are each independently selected from the group consisting of hydrogen, alkyl, alkenyl, acyl, and hydroxyalkyl;
R₃ is hydrogen or halogen;
the ring A is a benzene or pyridine ring;
the ring B is represented by the following formula (i) or (ii): 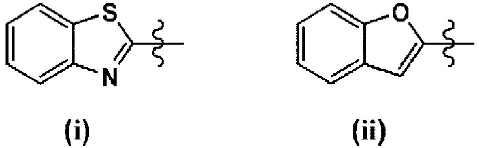
R₄ and R₅ are each independently selected from the group consisting of hydrogen, hydroxy, alkoxy, haloalkoxy, halohydroxyalkoxy, and aminoalkyl.

16. The α-synuclein aggregation inhibitor as set forth in claim 15, wherein the ring A is a pyridine ring.

17. The α-synuclein aggregation inhibitor as set forth in claim 15 or 16, wherein the ring B is represented by the formula (i).

18. The α-synuclein aggregation inhibitor as set forth in claim 15, wherein the compound represented by the formula (I) is selected from the following group:

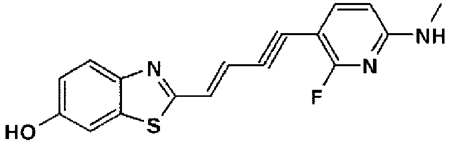

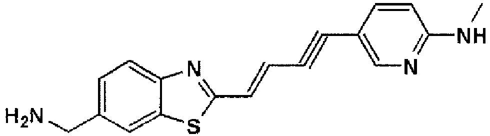

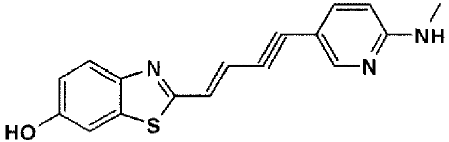

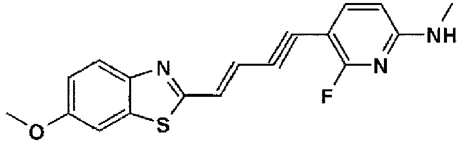

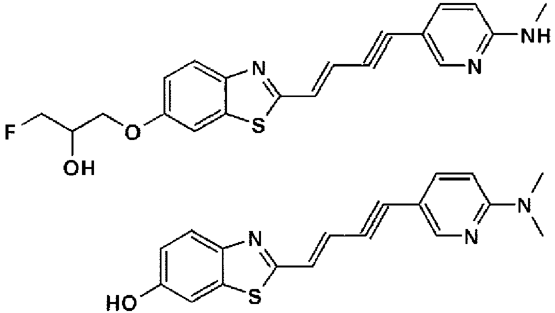

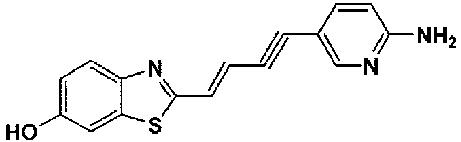

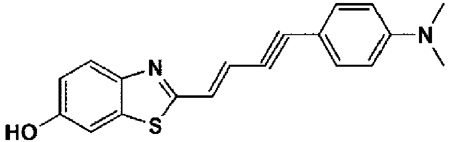

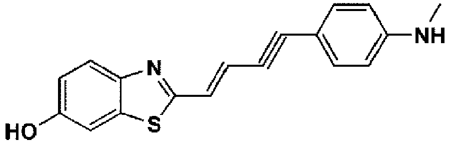

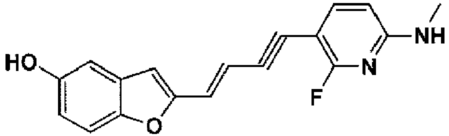

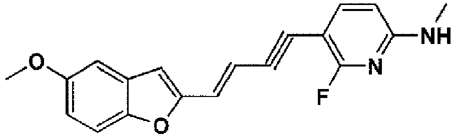

19. A compound represented by the following formula, a pharmaceutically acceptable salt thereof, or a solvate thereof:

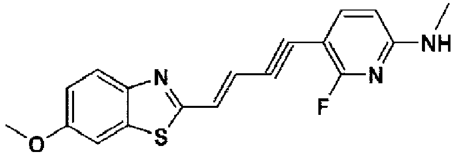

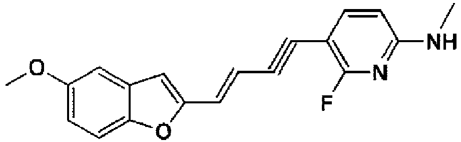

20. A compound represented by the following formula, a pharmaceutically acceptable salt thereof, or a solvate thereof: 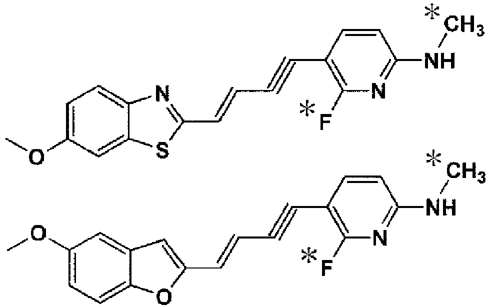 wherein at least one of atoms with the symbol * is a radioisotope thereof.

21. An intermediate for synthesizing a compound recited in claim 19, wherein said intermediate is represented by the following formula:

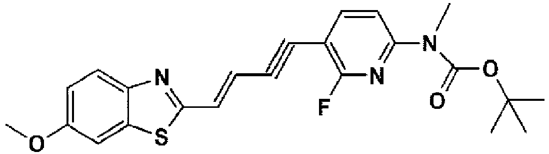

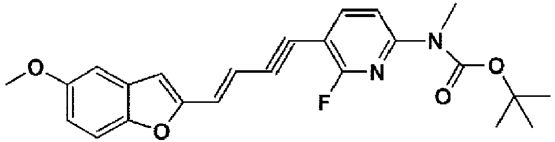

22. An intermediate for synthesizing a compound recited in claim 20, wherein said intermediate is represented by the following formula:
